# EUROPEAN PATENT APPLICATION

(11) **EP 2 495 304 A1**
(43) Date of publication of application: **05.09.2012**
(21) Application number: 10193713.4
(22) Date of filing: 03.12.2010
(51) Int. Cl.: C12N 1/16

(54) **Dicarboxylic acid production in a yeast cell**

(71) Applicant: DSM IP Assets B.V., 6411 TE Heerlen (NL)
(72) Inventor: Verwaal, René, 2631 NL, NOOTDORP (NL); Wu, Liang, 2628 LC, DELFT (NL); Wiessenhaan, Nathalie, 3039 KP, DELFT (NL); Nijkamp, Karin, 3081 RM, ROTTERDAM (NL)
(74) Representative: Cazemier, Anne Engeline

(57) **Abstract**

The present invention related to a recombinant yeast cell which comprises a genetic modification with/of at least one nucleotide sequence encoding an enzyme selected from the group consisting of a NAD(H)-dependent fumarate reductase, a fumarase, a malate dehydrogenase, a phosphoenolpyruvate (PEP) carboxykinase, a malate dehydrogenase, a pyruvate carboxylase, an isocitrate lyase, and a malate synthase, and wherein the yeast comprises a genetic modification with a nucleotide sequence encoding a dicarboxylic acid transporter from *Aspergillus niger.* The invention also relates to a process for producing a dicarboxylic acid comprising fermenting a recombinant yeast in a suitable fermentation medium.

## Description

The present invention relates to a recombinant yeast cell and a process for the production of a C4 dicarboxylic acid comprising fermenting the recombinant yeast cell.

The 4-carbon dicarboxylic acids malic acid, fumaric acid and succinic acid are potential precursors for numerous chemicals. For example, succinic acid can be converted into 1,4-butanediol (BDO), tetrahydrofuran, and gamma-butyrolactone. Another product derived from succinic acid is a polyester polymer which is made by linking succinic acid and BDO.

Succinic acid is predominantly produced through petrochemical processes by hydrogenation of butane. These processes are considered harmful for the environment and costly. The fermentative production of succinic acid is considered an attractive alternative process for the production of succinic acid, wherein renewable feedstock as a carbon source may be used.

Several studies have been carried out on the fermentative production of C4-dicarboxylic acid in (recombinant) yeast.

WO2009/065778 discloses a recombinant yeast suitable for succinic acid production, which had been genetically modified with a gene encoding a NAD(H) dependent fumarate reductase and for instance a gene encoding a heterologous fumarase or a dicarboxylic acid transporter, for instance a malic acid transporter from *Schizosaccharomyces pombe.* WO2007/061590, WO2008/144626 and WO2009/011974 disclose a pyruvate decarboxylase negative yeast suitable for malic acid production, which comprised a genetic modification with a dicarboxylic acid transporter for instance derived from *S*. *pombe* or *Aspergillus oryzae.*

Despite the improvements that have been made in the fermentative production of dicarboxylic acid in yeast, there remains a need for improved yeast cells for the fermentative production of dicarboxylic acids.

### Summary of the invention

The present invention relates to a recombinant yeast cell which comprises a genetic modification with at least one nucleotide sequence encoding an enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase, and wherein the yeast comprises a genetic modification with a nucleotide sequence encoding a dicarboxylic acid transporter from *Aspergillus niger.*

Surprisingly, a yeast cell according to the present disclosure produces a higher amount of a dicarboxylic acid than a yeast cell not comprising a genetic modification as disclosed herein.

The present invention further relates to a process for producing a dicarboxylic acid comprising fermenting a yeast cell in a suitable fermentation medium and producing the dicarboxylic acid.

### Definitions

Sequence identity is herein defined as a relationship between two or more amino acid (polypeptide or protein) sequences or two or more nucleic acid (polynucleotide) sequences, as determined by comparing the sequences. Usually, sequence identities or similarities are compared over the whole length of the sequences compared. In the art, "identity" also means the degree of sequence relatedness between amino acid or nucleic acid sequences, as the case may be, as determined by the match between strings of such sequences.

Preferred methods to determine identity are designed to give the largest match between the sequences tested. Methods to determine identity and similarity are codified in publicly available computer programs. The percent identity between two amino acid sequences or between two nucleotide sequences may be determined using the Needleman and Wunsch algorithm (Needleman, S. B. and Wunsch, C. D. (1970) J. Mol. Biol. 48, 443-453). Both amino acid sequences and nucleotide sequences can be aligned by the Needleman-Wunsch algorithm, which has been implemented in the computer program NEEDLE. For the purpose of this invention the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276 - 277, http://emboss.bioinformatics.nl/). For protein sequences EBLOSUM62 is used for the substitution matrix. For nucleotide sequence, EDNAFULL is used. The optional parameters used are a gap-open penalty of 10 and a gap extension penalty of 0.5. The skilled person will appreciate that all these different parameters will yield slightly different results but that the overall percentage identity of two sequences is not significantly altered when using different algorithms.

The nucleic acid and protein sequences of the present invention can further be used as a "query sequence" to perform a search against public databases to, for example, identify other family members or related sequences. Such searches can be performed using the nucleotide blast or protein blast programs available at NCBI (http://blastncbi.nlm.nih.gov/). For nucleic acid sequence searches the default parameters of the BLASTN algorithm can be used. For protein sequence searches the default parameters of the BLASTP algorithm can be used.

A recombinant yeast cell according to the present invention is defined herein as a cell which contains, or is transformed or genetically modified with a nucleotide sequence that does not naturally occur in the eukaryotic cell, or it contains additional copy or copies of an endogenous nucleic acid sequence, or it comprises a deletion or disruption of an endogenous nucleic acid sequence. A wild-type yeast cell is herein defined as the parental cell of the recombinant yeast cell.

A genetic modification with a nucleotide sequence is used herein to indicate that a gene or a nucleotide sequence is introduced into a (yeast) cell by any available means. A nucleotide sequence or gene may be prepared according to any method in the art, for instance extracted from an organism or synthesized by chemical means.

The term "homologous" or "endogenous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain.

The term "heterologous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous nucleic acids or proteins are not endogenous to the cell into which it is introduced, but have been obtained from another cell or synthetically or recombinantly produced.

The term "gene", as used herein, refers to a nucleic acid sequence containing a template for a nucleic acid polymerase, in yeasts, RNA polymerase II. Genes are transcribed into mRNAs that are then translated into protein.

The term "nucleic acid" as used herein, includes reference to a deoxyribonucleotide or ribonucleotide polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids). A polynucleotide can be full-length or a subsequence of a native or heterologous structural or regulatory gene. Unless otherwise indicated, the term includes reference to the specified sequence as well as the complementary sequence thereof.

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, glycosylation, lipid attachment, sulfation, gamma-carboxylation of glutamic acid residues, hydroxylation and ADP-ribosylation.

The term "enzyme" as used herein is defined as a protein which catalyses a (bio)chemical reaction in a cell.

To increase the likelihood that an introduced enzyme or protein is expressed in active form in a yeast cell of the invention, the corresponding encoding nucleotide sequence may be adapted to optimise its codon usage to that of the chosen yeast host cell. Several methods for codon optimisation are known in the art. A preferred method to optimise codon usage of the nucleotide sequences to a yeast cell is codon pair optimization technology as disclosed in WO2008/000632. Codon-pair optimization is a method for producing a polypeptide in a host cell, wherein the nucleotide sequences encoding the polypeptide have been modified with respect to their codon-usage, in particular the codon-pairs that are used, to obtain improved expression of the nucleotide sequence encoding the polypeptide and/or improved production of the polypeptide. Codon pairs are defined as a set of two subsequent triplets (codons) in a coding sequence.

Usually, a nucleotide sequence encoding an enzyme or protein, is operably linked to a promoter that causes sufficient expression of the corresponding nucleotide sequence.

As used herein, the term "operably linked" refers to a linkage of polynucleotide elements (or coding sequences or nucleic acid sequence) in a functional relationship. A nucleic acid sequence is "operably linked" when it is placed into a functional relationship with another nucleic acid sequence. For instance, a promoter or enhancer is operably linked to a coding sequence if it affects the transcription of the coding sequence.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences known to one of skilled in the art. A "constitutive" promoter is a promoter that is active under most environmental and developmental conditions. An "inducible" promoter is a promoter that is active under environmental or developmental regulation.

A promoter that could be used to achieve expression of a nucleotide sequence, may be not native to the nucleotide sequence to be expressed, i.e. a promoter that is heterologous to the nucleotide sequence (coding sequence) to which it is operably linked. Preferably, the promoter is homologous, i.e. endogenous to the host cell.

Suitable promoters in yeast cells are known to the skilled person in the art. Suitable promotors may be, but are not limited to TDH1, TDH3, GAL7, GAL10, GAL1, CYC1, HIS3, ADH1, PH05, ADC1, ACT1, TRP1, URA3, LEU2, ENO1, TPI1,. Other suitable promoters include PDC1, GPD1, PGK1, and TEF1.

Usually a nucleotide sequence encoding an enzyme or protein comprises a terminator. Any terminator, which is functional in the cell, may be used in the present invention. Preferred terminators are obtained from natural genes of the host cell. Suitable terminator sequences are well known in the art. Preferably, such terminators are combined with mutations that prevent nonsense mediated mRNA decay in the host cell of the invention (see for example: Shirley et al., 2002, Genetics 161:1465-1482).

An introduced nucleotide sequence may be overexpressed. It was found that an increased production of a dicarboxylic acid by a yeast cell may be achieved when a nucleotide sequence is overexpressed.

There are known methods in the art for overexpression of nucleotide sequences. A nucleotide sequence may be overexpressed by increasing the copy number of a gene or nucleotide sequence in a yeast cell, e.g. by integrating additional copies of the gene or nucleotide sequence in the cell's genome, by expressing the gene or nucleotide sequence from a centromeric vector, from an episomal multicopy expression vector or by introducing an (episomal) expression vector that comprises multiple copies of one or more gene(s). Overexpression of a nucleotide sequence encoding an enzyme or a protein may for instance be achieved with a (strong) constitutive promoter.

A nucleotide sequence may be ligated into a nucleic acid construct, for instance a plasmid, such as a low copy plasmid or a high copy plasmid. A yeast cell according to the present disclosure may comprise a single, but preferably comprises multiple copies of a nucleotide sequence for instance by multiple copies of a nucleotide construct.

A nucleic acid construct may be maintained episomally and thus comprises a sequence for autonomous replication, such as an autosomal replication sequence. A suitable episomal nucleic acid construct may e.g. be based on the yeast 2µ or pKD1 plasmids (Gleer et al., 1991, Biotechnology 9: 968-975), or the AMA plasmids (Fierro et al., 1995, Curr Genet. 29:482-489). Alternatively, each nucleic acid construct may be integrated in one or more copies into the genome of the yeast cell. Integration into the cell's genome may occur at random by non-homologous recombination but preferably, the nucleic acid construct may be integrated into the cell's genome by homologous recombination as is well known in the art.

### Detailed description

In one embodiment, a recombinant yeast cell comprising a genetic modification with at least one nucleotide sequence may encode any suitable homologous or heterologous enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase according the present disclosure comprises a nucleotide sequence encoding a dicarboxylic acid transporter from *Aspergillus niger,* which may have at least 80% sequence identity to SEQ ID NO: 2, for instance at least 85%, 90%, 95%, or 98, 99% sequence identity to SEQ ID NO: 2. A yeast cell according to the present disclosure may comprise a nucleotide sequence encoding a dicarboxylic acid transporter according to SEQ ID NO: 2.

A dicarboxylic acid transporter as used herein is defined as a protein capable of transporting a dicarboxylic acid across a cell membrane. A dicarboxylic acid transporter may transport a dicarboxylic acid in an ionic form and in a protonated form. A dicarboxylic acid transporter may transport other species, for instance a proton, together with a dicarboxylic acid. Transport as used herein may be import of a molecule from the exterior of a cell into a cell, and/or export of a molecule inside a cell to the exterior of a cell.

The at least one nucleotide sequence may encode any suitable homologous or heterologous enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase. An enzyme may be derived from any suitable microorganism.

An enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase in a yeast cell as disclosed herein may be localized in the cytosol. An amino acid sequence of an enzyme as disclosed herein may comprise a targeting signal, for instance a peroxisomal or mitochondrial targeting signal, for instance as determined by the method disclosed by Schlüter et al, Nucleic acid Research 2007, Vol 25, D815-D822. Deletion of targeting signal is expected to localize the protein in the cytosol. It was found that cytosolic localization of the at least one an enzyme resulted in increased dicarboxylic acid production, for instance increased succinic acid production.

A recombinant yeast cell may comprise a genetic modification with at least one nucleotide sequence encoding an enzyme which may be a NAD(H)-dependent fumarate reductase, for instance a NAD(H) dependent fumarate reductase which has at least 80% identity to SEQ ID NO: 13, for instance a NAD(H) dependent fumarate reductase which has at least 85%, 90%, 95% or 98% or 99% sequence identity with SEQ ID NO: 13. A NAD(H) dependent fumarate reductase (EC 1.3.1.6) is an enzyme that catalyses the conversion of fumaric acid to succinic acid. A recombinant yeast cell of the present disclosure may comprise a nucleotide sequence encoding a NAD(H)-dependent fumarate reductase of SEQ ID NO: 13. A NAD(H)-dependent fumarate reductase may be obtainable from any suitable microorganisms, for instance from *Trypanosoma brucei.*

A recombinant yeast cell of the present disclosure may comprise a genetic modification with/of at least one nucleotide sequence encoding an enzyme wherein the fumarase has at least 80% identity to SEQ IN NO: 14, for instance a fumarase which has at least 85%, 90%, 95% or 98% or 99% sequence identity with SEQ ID NO: 14. A fumarase (E.C. 4.2.1.2) is an enzyme that catalyses the conversion of malic acid to fumaric acid. A yeast cell of the present disclosure may comprise a nucleotide sequence encoding a fumarase of SEQ ID NO: 14. A fumarase may be obtainable from any suitable micro-organism, for instance *Rhizopus oryzae.*

A yeast cell according to present disclosure may comprise a genetic modification with at least one nucleotide sequence encoding an enzyme which may be a PEP-carboxykinase has at least 80% identity to SEQ ID NO: 15 for instance at least 85%, 90%, 95% or 98% or 99% sequence identity with SEQ ID NO: 15. A PEP-carboxykinase (E.C. 4.1.1.49) is an enzyme that catalyses the conversion of phosphoenolpyruvate to oxaloacetate. A recombinant yeast cell of the present disclosure may comprise a nucleotide sequence encoding a PEP-carboxykinase according to SEQ ID NO: 15. A PEP-carboxykinase may be obtainable from any suitable microorganism, for instance from *Actinobacillus succinogenes* or *Mannheimia succiniciproducens.*

A yeast cell according to the present disclosure may comprise a nucleotide sequence encoding a malate dehydrogenase which has at least 80% sequence identity with SEQ ID NO: 16, for instance at least 85%, 90%, 95% or 98% or 99% sequence identity with SEQ ID NO: 16. A malate dehydrogenase (E.C. 1.1.1.37) as used herein is as an enzyme that catalyses the conversion of oxaloacetate to malate. A recombinant yeast cell of the present disclosure may comprise a nucleotide sequence encoding a malate dehydrogenase according to SEQ ID NO: 16. A malate dehydrogenase may be obtainable from any suitable microorganism, for instance from *S*. *cerevisiae,* for instance Mdh2 or Mdh3.

A yeast cell according to the present disclosure may comprise a nucleotide sequence encoding a pyruvate carboxylase. which has at least 80% sequence identity with SEQ ID NO: 16, for instance at least 85%, 90%, 95% or 98% or 99% sequence identity with SEQ ID NO: 17. A pyruvate carboxylase (E.C. 6.4.1.1) is an enzyme that catalyses the conversion of pyruvate to oxaloacetate. A recombinant yeast cell of the present disclosure may comprise a nucleotide sequence encoding a pyruvate carboxylase according to SEQ. ID. NO:17. A pyruvate carboxylase may be obtainable from any suitable microorganism, for instance from *S*. *cerevisiae.*

A yeast cell according to the present disclosure may comprise a nucleotide sequence encoding an isocitrate lyase, for instance an isocitrate lyase that has at least 80% sequence identity with SEQ ID NO: 18, for instance at least 85%, 90%, 95% or 98% or 99% sequence identity with SEQ ID NO: 18. An isocitrate lyase (E.C. 4.1.3.1.) is an enzyme that catalyses the conversion of isocitrate to succinate and glyoxylate. A recombinant yeast cell of the present disclosure may comprise a nucleotide sequence encoding isocitrate lyase according to SEQ. ID. NO:18. An isocitrate lyase may be obtainable from any suitable microorganism, for instance *Kluyveromyces lactis.*

A yeast cell according to the present disclosure may comprise a nucleotide sequence encoding a malate synthase, for instance an malate synthase that has at least 80% sequence identity with SEQ ID NO: 19, for instance at least 85%, 90%, 95% or 98% or 99% sequence identity with SEQ ID NO: 19. A malate synthase (E.C. 2.3.3.9) is an enzyme that catalyses the conversion of glyoxylate and acetyl-CoA to malate. A malate synthase may be obtainable from any suitable microorganism, for instance from *S. cerevisiae.*

A recombinant yeast cell according to the present disclosure may be a cell wherein at least one gene encoding alcohol dehydrogenase is not functional. An alcohol dehydrogenase gene that is not functional is used herein to describe a yeast cell, which comprises a reduced alcohol dehydrogenase activity compared to a cell wherein all genes encoding an alcohol dehydrogenase are functional. A gene may become not functional by known methods in the art, for instance by mutation, disruption, or deletion, for instance by the method disclosed by Güldener et al., 1996, Nucleic Acids Res. 1996 Jul 1;24(13):2519-2524. In the event a yeast cell is a *Saccharomyces cerevisiae,* on.e or more genes encoding alcohol dehydrogenase, like *adh1* and/or *adh2,* are inactivated. Inactivation of an alcohol dehydrogenase was found to result in reduced ethanol production as compared to cell not comprising an inactivation of alcohol dehydrogenase.

A recombinant yeast cell according to the present disclosure may comprise at least one gene encoding glycerol-3-phosphate dehydrogenase which is not functional. A glycerol-3-phosphate dehydrogenase gene that is not functional is used herein to describe a yeast cell, which comprises a reduced glycerol-3-phosphate dehydrogenase activity, for instance by mutation, disruption, or deletion of the gene encoding glycerol-3-phosphate dehydrogenase, resulting in a decreased formation of glycerol as compared to the wild-type cell.

In one embodiment a recombinant yeast cell as disclosed herein may comprise a genetic modification with a nucleotide sequence encoding a NAD(H)-dependent fumarate reductase, a fumarase, a malate dehydrogenase, and/or a phosphoenolpyruvate (PEP) carboxykinase.

A recombinant yeast cell according to the present disclosure, may belong to the genera *Saccharomyces, Schizosaccharomyces, Issatchenkia, Pichia, Kluyveromyces, Yarrowia, Candida, Hansenula, Humicola, Zygosaccharomyces, Pachysolen* or *Yamadazyma.* A yeast cell may for instance belong to a species *Saccharomyces cerevisiae, Saccharomyces uvarum, Saccharomyces bayanus, Schizosaccharomyces pombe, Issatchenkia orientalis, Pichia stipidis, Kluyveromyces marxianus, K. lactis, K. thermotolerans, Yarrowia lipolytica, Candida sonorensis, C. glabrata, Hansenula polymorpha, Zygosaccharomyces bailii,* for instance *Saccharomyces cerevisiae.*

A yeast cell according to the present disclosure may be able to grow on any suitable carbon source known in the art and convert it to a desirable dicarboxylic acid as mentioned herein before. A yeast cell may be able to convert directly plant biomass, celluloses, hemicelluloses, pectines, or other C-sources such as rhamnose, galactose, fucose, maltose, maltodextrines, ribose, ribulose, or starch, starch derivatives, sucrose, lactose and glycerol. A recombinant yeast cell as used herein may comprise further genetic modification to be able to convert several C-sources. A recombinant yeast cell may express a nucleotide sequence encoding an enzyme selected from the group consisting of cellulases (endocellulases and exocellulases), hemicellulases (e.g. endo-and exo-xylanases, arabinases) necessary for the conversion of cellulose into glucose monomers and hemicellulose into xylose and arabinose monomers, and pectinases able to convert pectines into glucuronic acid and galacturonic acid or amylases to convert starch into glucose monomers. A yeast cell may be able to convert a carbon source selected from the group consisting of glucose, fructose, galactose, xylose, arabinose, sucrose, lactose, raffinose and glycerol.

In one aspect, the present disclosure relates to a method for transforming a yeast cell, comprising introducing at least one nucleotide sequence encoding an enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase, and a nucleotide sequence encoding a dicarboxylic acid transporter from *Aspergillus niger* into the yeast cell, wherein a transformed yeast cell is produced. As used herein, a transformed yeast cell is understood to be a recombinant yeast cell.

In one aspect the present disclosure relates to a process for producing a dicarboxylic acid comprising fermenting a yeast cell as disclosed herein in a suitable fermentation medium and producing the dicarboxylic acid.

A process according to the present disclosure may be carried out under aerobic and/or anaerobic conditions. Preferably, the process is carried out under anaerobic conditions or under micro-aerophilic or oxygen limited conditions. An anaerobic fermentation process is herein defined as a fermentation process run in the absence of oxygen or in which substantially no oxygen is consumed, for instance less than 5, 2.5 or 1 mmol/L/h, and wherein organic molecules serve as both electron donor and electron acceptors.

An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used. In an oxygen-limited fermentation process, oxygen is usually supplied in low amounts. A skilled person knows how to adapt the supply of oxygen in relation to a biomass concentration during fermentation in an oxygen-limited process.

Fermenting a recombinant yeast in a process for producing a dicarboxylic acid may comprise a growth phase, wherein yeast cells are grown until a desired cell density, and a production phase, wherein a dicarboxylic acid is produced in a desired amount. Fermentation conditions, such as the composition of a fermentation medium, pH or temperature, during a growth phase and a production phase may be different.

A suitable fermentation medium may comprise any suitable carbon source and nitrogen source. A suitable C-source may be glucose, galactose or other C-sources as defined herein above.

A process for producing a dicarboxylic acid according to the present disclosure may be carried out at any suitable pH between 1 and 8. The pH during fermentation may be between 2 and 7, for instance between 2.5 and 6, or between 3 and 5. The pH may be maintained at a desired pH level by adding a neutralizing agent, for instance potassium hydroxide or sodium hydroxide. The pH during a growth phase may be higher than during a production phase. The pH value during producing a dicarboxylic acid may decrease, for instance until a pH value of 2 to 3.5 has been reached.

A suitable temperature at which the process according to the present disclosure may be carried out may be between 5 and 60°C,for instance between 10 and 50°C, or between 15 and 35°C, or between 18°C and 30°C. The skilled person in the art knows the optimal temperatures for fermenting a specific yeast cell.

A dicarboxylic acid produced in a process according to the present disclosure may be succinic acid, fumaric acid or malic acid, for instance succinic acid. The wording dicarboxylic acid and dicarboxylate (eg. malate, fumarate, succinate) are used interchangeably herein. Dicarboxylic acid usually indicates a protonated (hydrogenated) or acid form of dicarboxylic acid and dicarboxylate an ionic or alkaline form of a dicarboxylic acid.

A process for producing a dicarboxylic acid according to the present disclosure may comprise recovering the dicarboxylic acid from a fermentation medium. Recovering a dicarboxylic acid from a fermentation medium may be carried out by a suitable method known in the art, for instance by crystallisation, ammonium precipitation or ion exchange technology.

A process for producing a dicarboxylic acid according the present disclosure may further comprise a step of converting the dicarboxylic acid into a pharmaceutical, cosmetic, food, feed, or chemical product. Succinic acid may for instance be further converted into a polymer, such as polybutylene succinate (PBS) or other suitable polymers, for instance a polyester polyol.

In one aspect the present invention relates to the use of a gene encoding a dicarboxylic acid transporter from *Aspergillus niger* for increasing dicarboxylic acid production by a recombinant yeast cell.

The present invention also relates to a process for the producing a dicarboxylic acid wherein a yeast cell is used as dicarboxylic acid producer, wherein a dicarboxylic acid transporter from *A. niger is* used to increase dicarboxylic acid production.

### Genetic modifications

Standard genetic techniques, such as overexpression of enzymes in the host cells, genetic modification of host cells, or hybridisation techniques, are known methods in the art, such as described in Sambrook and Russel (2001) "Molecular Cloning: A Laboratory Manual (3rd edition), Cold Spring Harbor Laboratory, Cold Spring Harbor Laboratory Press*,* or F. Ausubel et al, eds., "Current protocols in molecular biology", Green Publishing and Wiley Interscience, New York (1987).

### FIGURES

**Figure 1**: Plasmid map of pSUC060, containing the DCT_01 synthetic gene sequence.
**Figure 2**: Plasmid map of pSUC061, containing the DCT_02 synthetic gene sequence.
**Figure 3**: Plasmid map of pSUC062, containing the DCT_03 synthetic gene sequence.
**Figure 4**: Plasmid map of pSUC063, containing the DCT_04 synthetic gene sequence.
**Figure 5**: Plasmid map of pSUC067, containing the DCT_08 synthetic gene sequence.
**Figure 6**: Plasmid map of pSUC068, containing the DCT_09 synthetic gene sequence.
**Figure 7**: Average succinic acid yield in strain not containing deletions (CEN.PK113-6B background). Succinic acid yield (gram SA produced per gram galactose consumed) after 7 days of production was calculated and is depicted in the figure. The data represent the average succinic acid yield and of a succinic acid production experiment performed with three individual transformants. Error bars represent the standard deviation of the three individual transformants.
**Figure 8**: Average succinic acid yield in strain deleted for *adh1, adh2* and *gpd1* (SUC-267 background). Succinic acid yield (gram SA produced per gram galactose consumed) after 7 days of production was calculated and is depicted in the figure. The data represent the average succinic acid yield and of a succinic acid production experiment performed with three individual transformants. Error bars represent the standard deviation of the three individual transformants.

### EXAMPLES

### Example 1. Effect of overexpression of putative dicarboxylic acid transporters on succinic acid production in S. cerevisiae cells

### 1.1. Gene sequences

Using BLASTP, putative dicarboxylic acid transporters present in the NCBI non-redundant protein sequence database that are homologous to malate permease protein sequence of *Schizosaccharomyces pombe,* UniProt accession number P50537, were identified. Six sequences were selected derived from a variety of organisms and displaying different percentages of identity compared to malate permease protein sequence MAE1 of *Schizosaccharomyces pombe,* as determined using the NEEDLE program (Needleman and Wunsch algorithm, Needleman, S. B. and Wunsch, C. D. (1970) J. Mol. Biol. 48, 443-453) (Table 1).

**Table 1. Putative dicarboxylic acid transporters**

| **Code** | **Accession UniProt** | **Source organism** | **% Identity to MAE1** | **SEQ ID NO: (protein, synthetic gene)** |
|---|---|---|---|---|
| DCT_01 | A1 CG61 | *Aspergillus clavatus* | 19.8 | 1,7 |
| DCT_02 | A2R8T9 | *Aspergillus niger* | 30.2 | 2,8 |
| DCT_03 | Q8X021 | *Neurospora crassa* | 23.9 | 3,9 |
| DCT_04 | B6K1M9 | *Schizosaccharomyce s japonicus* | 42.6 | 4,10 |
| DCT_08 | A6VN82 | *Actinobacillus succinogenes* | 10.4 | 5,11 |
| DCT_09 | Q8G3I8 | *Bifidobacterium longum* | 9.4 | 6,12 |

The nucleotide sequences are described as follows:
SEQ ID NO: 1, UniProt accession number A1CG61, described as C4-dicarboxylate transporter/malic acid transport protein, putative, from *Aspergillus clavatus.*
SEQ ID NO: 2, UniProt accession number A2R8T9, described as putative dicarboxylate transporter/malic acid transport protein, from *Aspergillus niger.*
SEQ ID NO: 3, UniProt accession number Q8X021, described as putative dicarboxylate transporter/malic acid transport protein, from *Neurospora crassa.*
SEQ ID NO: 4, UniProt accession number B6K1M9, described as malic acid transport protein, from *Schizosaccharomyces japonicus.*
SEQ ID NO: 5, UniProt accession number A6VN82, described as anaerobic c4-dicarboxylate antiporter, DcuC family, from *Actinobacillus succinogenes.*
SEQ ID NO: 6, UniProt accession number Q8G3I8, described as C4-dicarboxylate transporter, from *Bifidobacterium longum.*
SEQ ID NO: 1, 2, 3, 4, 5 and 6 were codon-pair optimized for expression in S. *cerevisiae* according to a method as disclosed in WO2008/000632.

In all synthetic gene sequences, named DCT_01, DCT_02, DCT_03, DCT_04, DCT_08 and DCT_09, the stop codon was modified to TAAG. The synthetic DCT genes are under control of (or operable linked to) a strong promoter from S. *cerevisiae,* i.e. the ENO1-promote (600 bp upstream of the start codon of the *ENO1* gene). In the ENO1 promoter, T at position 596 (-5) was changed to A in order to obtain a better Kozak sequence. Proper termination is controlled by a terminator sequence from *S*. *cerevisiae,* i.e. the ENO1-terminator (300 bp downstream of the stop codon of the PGK1 gene). The ENO1-promoter; DCT-gene; ENO1-terminator sequences were surrounded by unique restriction enzymes sites including *Bam*HI at the 5' end and *Notl* at the 3' end of the synthetic gene sequences. The resulting sequences SEQ ID NO: 7 (DCT_01), SEQ ID NO: 8 (DCT_02), SEQ ID NO: 9 (DCT_03), SEQ ID NO: 10 (DCT_04), SEQ ID NO: 11 (DCT_08) and SEQ ID NO: 12 (DCT_09) were synthesized by Geneart (Regensburg, Germany).

### 1.2. Construction of expression vectors

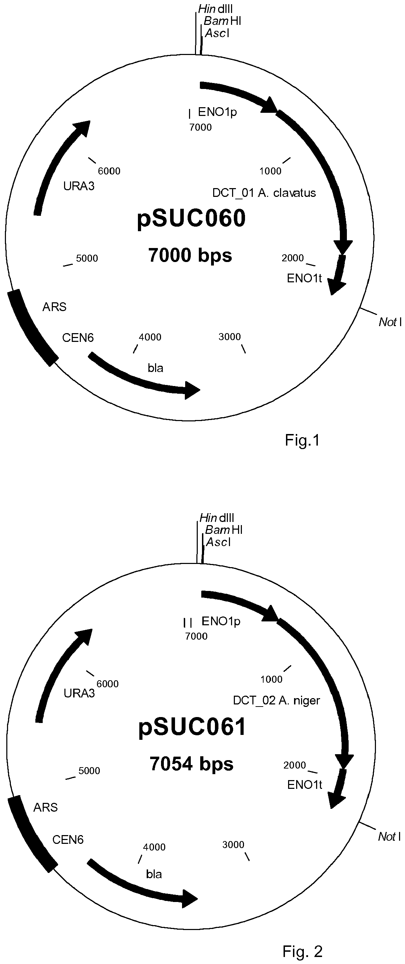

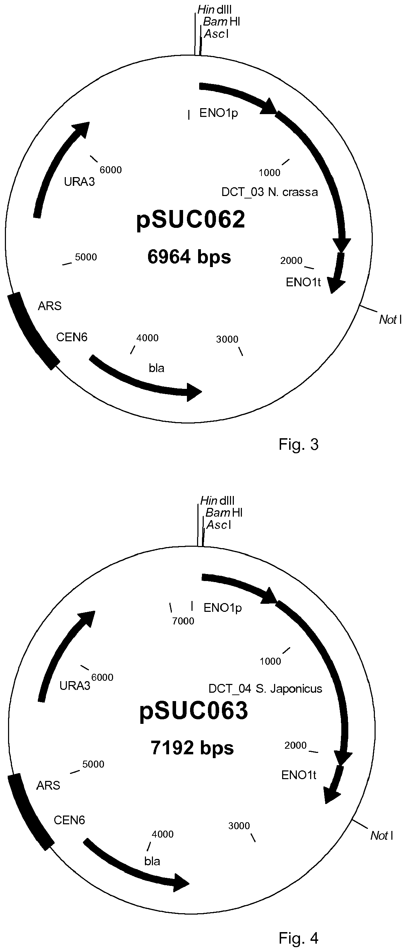

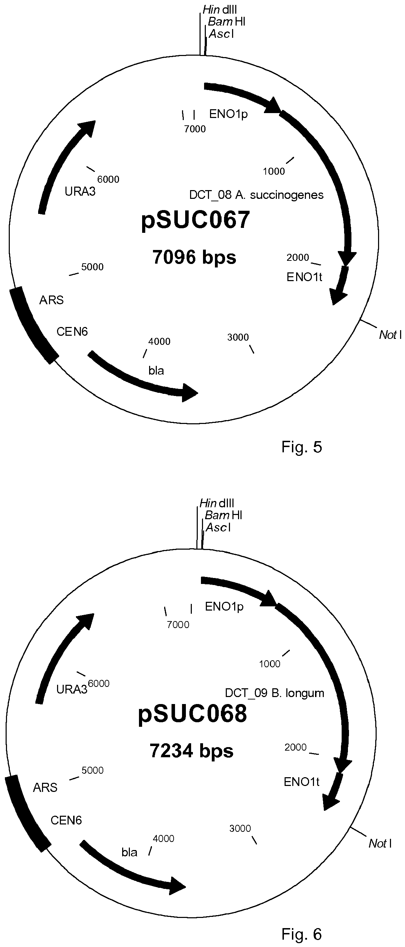

All synthetic genes were restricted with the restriction enzymes *Bam*HI and *Not*l and the nucleotide sequences were ligated into *S*. *cerevisiae* expression vector pRS416 (Sirkoski R.S. and Hieter P., Genetics, 1989, 122(1):19-27) that was restricted with the restriction enzymes *Bam*HI and *Not*l. pSUC060 (DCT_01, Figure 1), pSUC061 (DCT_02, Figure 2), pSUC062 (DCT_03, Figure 3), pSUC063 (DCT_04, Figure 4), pSUC067 (DCT_08, Figure 5) and pSUC068 (DCT_09, Figure 6).

Construction of expression plasmids pGBS414PPK-3 and pGBS415FUM-3 were prepared as disclosed in WO2009/065778 p. 23-34 including the corresponding sequence listings, which is herein enclosed by reference.

### 1.3. S. cerevisiae strains

Strain SUC-267 was created as follows: Strain RWB064 (CEN.PK113-6B, *MATA ura3-52 leu2-112 trp1-289 adh1::loxP adh2::loxP gpd1::KanMXloxP*) was transformed with plasmid pSH47 for expression of Cre-recombinase in order to remove the KanMX marker flanked by loxP sites which was used for deletion of the *GPD1* gene. After transformants were selected for loss of the KanMX marker, the pSH47 plasmid was rescued, resulting in strain SUC-267 (CEN.PK113-6B, *MATA ura3-52 leu2-112 trp1-289 adh1::loxP adh2::loxP gpd1::loxP).* All procedures for removal of a KanMX marker flanked by loxP sites and rescue of plasmid pSH47 are described by Güldener et al., 1996, Nucleic Acids Res. 1996 Jul 1;24(13):2519-24 and were performed accordingly.

Plasmids pGBS414PPK-3, pGBS415FUM-3 and one of the plasmids pSUC060, pSUC061, pSUC062, pSUC063, pSUC067 and pSUC-068 (see Table 2) were transformed either into strain CEN.PK113-6B (*MATA ura3-52 leu2-112 trp1-289*) or into strain SUC-267, resulting in the strains listed in Table 2. The following control strains were created: CEN.PK113-6B and SUC-267 were each transformed with pGBS414PPK-3, pGBS415FUM-3 and pRS416 to create control strains containing 4 reductive TCA cycle genes, but no dicarboxylic acid transporter genes. In addition CEN.PK113-6B and SUC-267 were each transformed with pRS414, pRS415 and pRS416 (Sirkoski R.S. and Hieter P., Genetics, 1989, 122(1):19-27) to create control strains not overexpressing reductive TCA cycle or dicarboxylic acid transporter genes (Table 2).

The expression vectors were transformed into yeast by electroporation. The transformation mixtures were plated on Yeast Nitrogen Base (YNB) w/o AA (Difco) + 2% galactose. From the colonies appearing on the transformation plates, 6 were re-streaked and 3 individual transformants were checked for production of succinic acid.

**Table 2: List of yeast strains. All strains are prototrophic.**

| Name | Background | Plasmids | Genes |
|---|---|---|---|
| SUC-275 | CEN.PK113-6B | pGBS414PPK-3 | PCKa, FRDg |
| | | pGBS415FUM-3 | FUMR, MDH3 |
| | | pSUC060 | DCT_01 |
| SUC-276 | CEN.PK113-6B | pGBS414PPK-3 | PCKa, FRDg |
| | | pGBS415FUM-3 | FUMR, MDH3 |
| | | pSUC061 | DCT_02 |
| SUC-277 | CEN.PK113-6B | pGBS414PPK-3 | PCKa, FRDg |
| | | pGBS415FUM-3 | FUMR, MDH3 |
| | | pSUC062 | DCT_03 |
| SUC-278 | CEN.PK113-6B | pGBS414PPK-3 | PCKa, FRDg |
| | | pGBS415FUM-3 | FUMR, MDH3 |
| | | pSUC063 | DCT_04 |
| SUC-280 | CEN.PK113-6B | pGBS414PPK-3 | PCKa, FRDg |
| | | pGBS415FUM-3 | FUMR, MDH3 |
| | | pSUC067 | DCT_08 |
| SUC-281 | CEN.PK113-6B | pGBS414PPK-3 | PCKa, FRDg |
| | | pGBS415FUM-3 | FUMR, MDH3 |
| | | pSUC068 | DCT_09 |
| SUC-287 | CEN.PK113-6B | pGBS414PPK-3 | PCKa, FRDg |
| | | pGBS415FUM-3 | FUMR, MDH3 |
| | | pRS416 | No transporter |
| CEN.PK empty vector | CEN.PK113-6B | pRS414 | |
| | | pRS415 | |
| | | pRS416 | |
| SUC-290 | SUC-267 (*ΔADH1*, *ΔADH2, ΔGPD1)* | pGBS414PPK-3 | PCKa, FRDg |
| | | pGBS415FUM-3 | FUMR, MDH3 |
| | | pSUC060 | DCT_01 |
| SUC-291 | SUC-267 (*ΔADH1, ΔADH2, ΔGPD1)* | pGBS414PPK-3 | PCKa, FRDg |
| | | pGBS415FUM-3 | FUMR, MDH3 |
| | | pSUC061 | DCT_02 |
| SUC-292 | SUC-267 (*ΔADH1, ΔADH2, ΔGPD1)* | pGBS414PPK-3 | PCKa, FRDg |
| | | pGBS415FUM-3 | FUMR, MDH3 |
| | | pSUC062 | DCT_03 |
| SUC-293 | SUC-267 (*ΔADH1, ΔADH2, ΔGPD1)* | pGBS414PPK-3 | PCKa, FRDg |
| | | pGBS415FUM-3 | FUMR, MDH3 |
| | | pSUC063 | DCT_04 |
| SUC-295 | SUC-267 (*ΔADH1, ΔADH2, ΔGPD1)* | pGBS414PPK-3 | PCKa, FRDg |
| | | pGBS415FUM-3 | FUMR, MDH3 |
| | | pSUC067 | DCT_08 |
| SUC-296 | SUC-267 (*ΔADH1, ΔADH2, ΔGPD1)* | pGBS414PPK-3 | PCKa, FRDg |
| | | pGBS415FUM-3 | FUMR, MDH3 |
| | | pSUC068 | DCT_09 |
| SUC-302 | SUC-267 (*ΔADH1, ΔADH2, ΔGPD1)* | pGBS414PPK-3 | PCKa, FRDg |
| | | pGBS415FUM-3 | FUMR, MDH3 |
| | | pRS416 | No transporter |
| SUC-267 empty vector | SUC-267 (*ΔADH1, ΔADH2, ΔGPD1)* | pRS414 | |
| | | pRS415 | |
| | | pRS416 | |

### 1.4. Growth experiments and succinic acid production in wildtype CEN.PK strains

Transformants were inoculated in 25 ml pre-culture medium consisting of Verduyn medium (Verduyn et al., 1992, Yeast. Jul;8(7):501-17) comprising 1% or 2% galactose (w/v) for knockout or wildtype background respectively and grown under aerobic conditions in 100 ml shake flasks in a shaking incubator at 30°C at 280 rpm. After 48 hours 25 ml production medium was added. The production medium consisted of Verduyn medium with 5% or 20% galactose (w/v) respectively for the *adh1, adh2, gpd1* knockout (SUC-267) and wildtype (CEN.PK113-6B) background, supplemented with 1% CaCO3 (w/v). The cultures were incubated in a shaking incubator at 30°C at 100 rpm. Three and five days after inoculation galactose was added (50 g/L for the CEN.PK and 25 g/L for the SUC-267 background). In total 85 g/L galactose was added to the SUC-267 and 270 g/L galactose to the CEN.PK113-6B cultures.

After 7 days of cultivation, samples for flow NMR were prepared as follows: From each shake flask, 600 microliter culture was taken and centrifuged for 1 minute at 14,000 rpm. The supernatant was transferred to a 96-deep well MTP plate with a ratio 9 to 1 internal standard (20 g/L maleic acid, 40 g/I EDTA in D2O), with a minimum of 1000 microliters per well. If needed, dilutions were made to the end volume by adding to each well a mixture of 80:20 H₂O/D₂O containing DSS. Dicarboxylic acid concentrations and sugars in the fermentation supernatant were determined with a Bruker BEST avance II 500 MHz spectrometer. The NMR spectra were recorded at 27 degrees Celsius with a relaxation delay of 30 s.

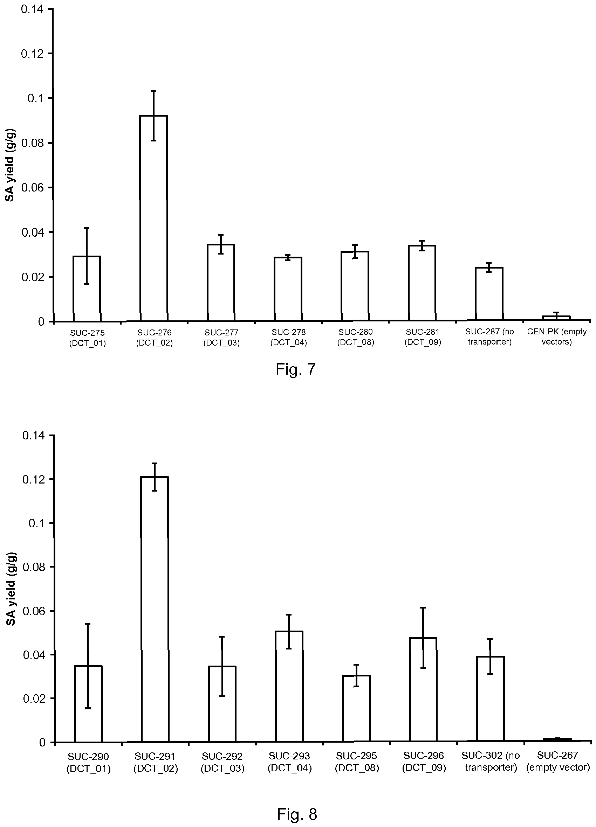

Succinic acid yields observed for the CEN.PK strain (CEN.PK113-6B background) and the strain deleted for *adh1, adh2* and *gpd1* (SUC-267 background) are depicted in Figures 7 and 8 respectively. The results show that overexpression of the putative dicarboxylic acid transporter designated DCT_02 (sequence from *A. niger,* UniProt Accession No. A2R8T9), in combination with PCKa, MDH3, FUMR, and FRDg, resulted in increased yields (SUC-276 in CEN.PK, SUC-291 in SUC-267 background) as compared to strains that did not overexpress any putative dicarboxylic acid transporter sequence (SUC-287 in CEN.PK, SUC-302 in SUC-267 background). In strains that overexpressed the putative transporter sequences DCT_01, DCT_03, DCT_04, DCT_08 or DCT_09 in combination with PCKa, MDH3, FUMR and FRDg, both in the CEN.PK and SUC-267 background, an improvement in yield as compared to control strains overexpressing no putative dicarboxylic acid sequence was not observed. Similar results were obtained both in a wildtype (CEN.PK113-6B background) and *adh1, adh2* and *gpd1* (SUC-267 background). These results indicate that, a dicarboxylic acid transporter t from *A. niger* can be used for export of succinic acid to the exterior of *S*. *cerevisiae* cells.

## Claims

1. A recombinant yeast cell which comprises a genetic modification with at least one nucleotide sequence encoding an enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase, and wherein the yeast comprises a genetic modification with a nucleotide sequence encoding a dicarboxylic acid transporter from *Aspergillus niger.*

2. A yeast cell according to claim 1, wherein the yeast cell upon expression of at least one nucleotide sequence and / or the nucleotide sequence encoding a dicarboxylic acid transporter from *A. niger,* produces a higher amount of a dicarboxylic acid than a yeast cell not comprising the genetic modification.

3. A yeast cell according to claim 1 or 2, wherein the dicarboxylic acid transporter has at least 80% sequence identity to SEQ ID NO: 2.

4. A yeast cell according to any one of the claims 1 to 3, wherein the enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase is localized in the cytosol.

5. A yeast cell according to any one of the claims 1 to 4, wherein the NAD(H) dependent fumarate reductase has at least 80% identity to SEQ ID NO: 13.

6. A yeast cell according to any one of the claims 1 to 5, wherein the fumarase has at least 80% identity to SEQ IN NO: 14.

7. A yeast cell according to any one of the claims 1 to 6, wherein the PEP-carboxykinase has at least 80% identity to SEQ ID NO: 15

8. A yeast cell according to any one of the claims 1 to 7, wherein the malate dehydrogenase has at least 80% identity to SEQ ID NO: 16.

9. A yeast cell according to any one of the claims 1 to 8, wherein the pyruvate carboxylase has at least 80% sequence identity to SEQ ID NO: 17.

10. A yeast cell according to any one of the claims 1 to 9, wherein the isocitrate lyase has at least 80% sequence identity to SEQ ID NO: 18.

11. A yeast cell according to any one of the claims 1 to 10, wherein the malate synthase has at least 80% sequence identity to SEQ ID NO: 19.

12. A yeast cell according to any one of the claims 1 to 11, wherein the yeast belongs to the genus *Saccharomyces, Kluyveromyces, Yarrowia,* or *Pichia.*

13. A yeast cell according to any on the claims 1 to 12, wherein the yeast is a *Saccharomyces cerevisiae.*

14. A method for transforming a yeast cell, comprising introducing at least one nucleotide sequence encoding an enzyme selected from the group consisting of a NAD(H)-dependent fumarate reductase, a fumarase, a malate dehydrogenase, a phosphoenolpyruvate (PEP) carboxykinase, a pyruvate carboxylase, an isocitrate lyase, and a malate synthase, and a nucleotide sequence encoding a dicarboxylic acid transporter from *Aspergillus niger* into the yeast cell, wherein a transformed yeast cell is produced.

15. A process for producing a dicarboxylic acid comprising fermenting a yeast cell according to any one of the claims 1 to 13 in a suitable fermentation medium and producing the dicarboxylic acid.

16. Process according to claim 15, wherein the dicarboxylic acid is succinic acid, fumaric acid, or malic acid.

17. Process according to claim 15 or 16, further comprising recovering the dicarboxylic acid from the fermentation medium.

18. Use of a gene encoding a dicarboxylic acid transporter from *Aspergillus niger for* increasing dicarboxylic acid production by a recombinant yeast cell.

19. A process for the producing a dicarboxylic acid wherein a yeast cell is used as dicarboxylic acid producer, wherein a dicarboxylic acid transporter from *A. niger* is used to increase dicarboxylic acid production.


SEQUENCE LISTING

<110>  DSM IP Assets B.V.

<120>  Dicarboxylic acid production in a yeast cell

<130>  27939-EP-EPA

<160>  19

<170>  PatentIn version 3.5

<210>  1
<211>  398
<212>  PRT
<213>  Aspergillus clavatus

<400>  1

Met Glu Arg Thr Leu Cys Pro Asn Thr Thr Pro Pro Ala Gln Glu Leu
1               5                   10                  15

Thr Lys His Asp Val Gly Trp Arg Arg Ile Val Arg Asn Phe Thr Pro
            20                  25                  30

Ser Trp Phe Ser Val Thr Met Gly Thr Gly Ile Val Ser Ile Leu Leu
        35                  40                  45

Asn Thr Leu Pro Tyr Asn Ala Pro Trp Leu Tyr Trp Ile Ser Val Val
    50                  55                  60

Leu Phe Ala Val Asn Val Leu Leu Phe Gly Thr Gly Cys Ile Ile Ser
65                  70                  75                  80

Val Leu Arg Tyr Ile Leu Tyr Pro Glu Val Phe Thr Ala Met Ile Ser
                85                  90                  95

His Pro Val Gln Ser Met Phe Ile Gly Thr Phe Pro Met Gly Leu Ala
            100                 105                 110

Thr Ile Ile Asn Met Phe Cys Phe Val Cys Val Pro Val Trp Gly Glu
        115                 120                 125

Trp Thr Arg Asn Cys Ala Trp Gly Leu Trp Ile Val Asp Ala Val Leu
    130                 135                 140

Ser Val Val Val Ala Leu Ser Leu Pro Phe Leu Leu Met Ala Asn Gly
145                 150                 155                 160

His Glu Thr Gln Leu Ser Ser Met Thr Ala Val Trp Leu Leu Pro Val
                165                 170                 175

Val Ser Cys Val Val Ala Ala Ser Ser Gly Ala Ile Val Ala Asp Ile
            180                 185                 190

Leu Pro Asn Pro Gln His Ala Leu Trp Thr Val Ile Val Ser Tyr Ile
        195                 200                 205

Leu Trp Gly Ile Gly Val Pro Leu Ala Leu Met Val Met Val Ile Tyr
    210                 215                 220

Leu Gln Arg Leu Thr Leu His Lys Leu Pro Pro Lys Ala Val Ile Val
225                 230                 235                 240

Ser Val Phe Leu Pro Leu Gly Pro Leu Gly Gln Gly Gly Tyr Gly Ala
                245                 250                 255

Met Lys Leu Gly Gln Ala Ala Gln Ser Ile Phe Pro Gln Thr His Thr
            260                 265                 270

Leu Asp Ala Ser Ala Gly Pro Met Phe Tyr Thr Leu Gly Phe Leu Val
        275                 280                 285

Ala Leu Ile Leu Trp Ser Phe Gly Leu Val Trp Leu Phe Phe Ala Ser
    290                 295                 300

Ala Ser Ile Ala Arg His Arg Asn Phe Pro Phe Asn Ile Gly Trp Trp
305                 310                 315                 320

Gly Phe Thr Phe Pro Leu Gly Val Phe Ala Thr Ser Thr Cys Gln Met
                325                 330                 335

Gly Arg Glu Leu Pro Ser Glu Phe Phe Arg Val Leu Gly Thr Ile Leu
            340                 345                 350

Ser Leu Cys Val Val Val Leu Trp Met Val Val Ser Met Gly Thr Leu
        355                 360                 365

Lys Gly Val Val Ser Gly Gln Leu Phe Phe Ala Pro Cys Leu Ala Asp
    370                 375                 380

Leu Lys Met Lys Glu Ala Asp Gly Asn Gly Glu Lys Arg Ala
385                 390                 395

<210>  2
<211>  416
<212>  PRT
<213>  Aspergillus niger

<400>  2

Met Asn Val Glu Thr Ser Leu Pro Gly Ser Ser Gly Ser Asp Leu Glu
1               5                   10                  15

Thr Phe His His Glu Thr Lys Lys His Ala Asn His Asp Ser Gly Ile
            20                  25                  30

Ser Val Asn His Glu Ala Glu Ile Gly Val Asn His Thr Phe Glu Lys
        35                  40                  45

Pro Gly Pro Val Gly Ile Arg Glu Arg Leu Arg His Phe Thr Trp Ala
    50                  55                  60

Trp Tyr Thr Leu Thr Met Ser Cys Gly Gly Leu Ala Leu Leu Ile Val
65                  70                  75                  80

Asn Gln Pro His Asp Phe Lys Gly Leu Lys Asp Ile Ala Arg Val Val
                85                  90                  95

Tyr Cys Leu Asn Leu Ala Phe Phe Val Ile Val Thr Ser Leu Met Ala
            100                 105                 110

Ile Arg Phe Ile Leu His Lys Asn Met Trp Glu Ser Leu Gly His Asp
        115                 120                 125

Arg Glu Gly Leu Phe Phe Pro Thr Phe Trp Leu Ser Ile Ala Thr Met
    130                 135                 140

Ile Thr Gly Leu Tyr Lys Cys Phe Gly Asp Asp Ala Asn Glu Lys Phe
145                 150                 155                 160

Thr Lys Cys Leu Gln Val Leu Phe Trp Ile Tyr Cys Gly Cys Thr Met
                165                 170                 175

Ile Thr Ala Val Gly Gln Tyr Ser Phe Val Phe Ala Thr His Lys Tyr

180                 185                 190

Glu Leu His Thr Met Met Pro Ser Trp Ile Leu Pro Ala Phe Pro Val
        195                 200                 205

Met Leu Ser Gly Thr Ile Ala Ser Val Ile Gly Ser Gly Gln Pro Ala
    210                 215                 220

Ser Asp Gly Ile Pro Ile Ile Ile Ala Gly Ile Thr Phe Gln Gly Leu
225                 230                 235                 240

Gly Phe Ser Ile Ser Phe Met Met Tyr Ala His Tyr Ile Gly Arg Leu
                245                 250                 255

Met Glu Val Gly Leu Pro Ser Pro Glu His Arg Pro Gly Met Phe Ile
            260                 265                 270

Cys Val Gly Pro Pro Ala Phe Thr Ala Leu Ala Leu Val Gly Met Ala
        275                 280                 285

Lys Ala Leu Pro Asp Asp Phe Gln Ile Val Gly Asp Pro His Ala Val
    290                 295                 300

Ile Asp Gly Arg Val Met Leu Phe Leu Ala Val Ser Ala Ala Ile Phe
305                 310                 315                 320

Leu Trp Ala Leu Ser Phe Trp Phe Phe Cys Ile Ala Val Val Ala Val
                325                 330                 335

Val Arg Ser Pro Pro Lys Gly Phe His Leu Asn Trp Phe Ala Met Val
            340                 345                 350

Phe Pro Asn Thr Gly Phe Thr Leu Ala Thr Ile Thr Leu Ala Asn Met
        355                 360                 365

Phe Glu Ser Pro Gly Val Lys Gly Val Ala Thr Ala Met Ser Leu Cys
    370                 375                 380

Val Ile Ile Met Phe Ile Phe Val Leu Val Ser Ala Ile Arg Ala Val
385                 390                 395                 400

Ile Arg Lys Asp Ile Met Trp Pro Gly Gln Asp Glu Asp Val Ser Glu
                405                 410                 415

<210>  3
<211>  386
<212>  PRT
<213>  Neurospora crassa

<400>  3

Met Lys Ala Ser Met Asp Asp Ile Leu Thr Lys Gly Arg Asn Leu Ser
1               5                   10                  15

Lys Gly Lys Val Ser Leu Arg Asp Arg Ile Thr Cys Tyr Gln Trp Thr
            20                  25                  30

Trp Phe Thr Met Thr Met Ala Thr Gly Gly Val Ala Asn Val Leu His
        35                  40                  45

Ser Ile Pro Tyr Lys Ser Gln Trp Leu Thr Gly Val Gly Leu Phe Phe
    50                  55                  60

Phe Phe Leu Asn Leu Val Leu Phe Thr Val Asn Cys Ile Cys Ile Thr
65                  70                  75                  80

Leu Arg Phe Val Trp Ser Arg Gly Ser Phe Leu His Ser Leu Asn Asp
                85                  90                  95

Gln Thr Glu Ser Leu Phe Val Ala Gly Thr Ile Phe Ile Thr Ile Cys
            100                 105                 110

Gln Tyr Gly Ile Pro Asn Thr Gly Pro Trp Leu Leu Lys Ala Met Glu
        115                 120                 125

Ile Ile Phe Trp Ile Tyr Val Gly Leu Ser Phe Ser Ile Ser Ala Phe
    130                 135                 140

Leu Tyr Leu Leu Leu Trp Ser Thr Thr Val Phe Pro Ile His Thr Met
145                 150                 155                 160

Thr Pro Val Trp Val Phe Pro Ala Tyr Pro Leu Leu Leu Thr Ala Pro
                165                 170                 175

Tyr Gly Ala Asn Leu Ile Ala Ala Ala Val Lys Ser Gly His Leu Thr
            180                 185                 190

Arg Ala Gly Gln Ser Thr Ile Asn Pro Ile Ser Ile Ala Leu Ala Ser
        195                 200                 205

Val Ala Val Gln Gly Thr Gly Phe Leu Ile Ser Phe Met Ile Cys Ala
    210                 215                 220

Ala Phe Leu Tyr Arg Leu Met Thr Gln Lys Leu Pro Arg Asp Tyr Gln
225                 230                 235                 240

Arg Pro Gly Val Phe Ile Ser Ile Gly Pro Gly Ala Phe Thr Ala Ala
                245                 250                 255

Gly Leu Val Gln Leu Ala Asn Ser Ala Pro Asp Phe Phe Pro Thr Glu
            260                 265                 270

Phe Leu Gly Thr Gln Gly Ala Val Ala Ile Ser Ile Leu Arg Met Leu
        275                 280                 285

Ala Tyr Met Ala Gly Ile Trp Leu Trp Gly Leu Ser Cys Trp Phe Phe
    290                 295                 300

Leu Val Ser Val Gly Ser Leu Trp Lys Tyr Leu Arg Pro Glu Arg Lys
305                 310                 315                 320

Ser Lys Leu Gln Phe Gln Met Thr Trp Phe Ser Phe Val Phe Pro Asn
                325                 330                 335

Thr Ala Leu Val Thr Ala Thr Glu Gln Leu Gly Lys Ala Phe Glu Ser
            340                 345                 350

Asp Gly Leu Lys Ile Leu Gly Cys Val Leu Thr Gly Cys Ile Ile Val
        355                 360                 365

Val Trp Ile Val Val Phe Trp Arg Met Leu Glu Cys Ile Trp Arg Arg
    370                 375                 380

Glu Leu
385

<210> 4
<211> 462
<212> PRT
<213> Schizosaccharomyces japonicus

<400>  4

Met Val Pro Pro Leu Leu Lys Gln Arg Tyr His Glu Leu Phe Asp Leu
1               5                   10                  15

Arg Val Arg Gly Pro Arg Ile Pro Leu Lys Lys Arg Leu Glu Cys Phe
            20                  25                  30

Ser Trp Ala Trp Phe Ala Ser Ser Met Gly Thr Gly Gly Ile Ala Met
        35                  40                  45

Val Ile Ser Leu Tyr Pro Tyr Arg Phe Tyr Gly Met Asn Thr Ile Gly
    50                  55                  60

Lys Val Val Phe Ile Phe Gln Leu Cys Leu Leu Ala Thr Tyr Phe Ile
65                  70                  75                  80

Cys Ile Thr Tyr Arg Phe Val Arg Val Pro Gly Ser Leu Gln Lys Ser
                85                  90                  95

Trp Thr Asn Pro Ala Glu Met Leu Phe Ile Pro Thr Ser Leu Leu Ser
            100                 105                 110

Val Ala Thr Ser Ile Ser Cys Leu Asp Pro Phe Ala Trp Pro Tyr Val
        115                 120                 125

Gly Ile Trp Met Val Tyr Phe Ile Arg Val Val Tyr Trp Ile Phe Val
    130                 135                 140

Gly Val Ser Phe Ile Phe Val Met Gly Gln Phe Tyr Thr Leu Tyr Ser
145                 150                 155                 160

Arg Asn Ile Phe Thr Pro Glu Thr Ile Ala Pro Ala Leu Val Leu Pro
                165                 170                 175

Ile Phe Pro Cys Met Ile Cys Gly Val Ile Ala Ser Ala Ile Ala Glu
            180                 185                 190

Ser Gln Pro Ala Lys Gln Ala Lys Asp Met Val Ile Ala Gly Ile Ala
        195                 200                 205

Phe Gln Gly Leu Gly Phe Trp Val Tyr Ile Met Val Tyr Ala Val Asn
    210                 215                 220

Met Tyr Lys Leu Phe Ile Tyr Gly Leu Met Pro Ala Ser Asp Arg Pro
225                 230                 235                 240

Gly Leu Phe Ile Phe Val Ser Pro Pro Ser Phe Thr Gly Leu Thr Leu
                245                 250                 255

Leu Asp Leu Ser Phe Ala Ala Arg Lys Lys Arg Pro Tyr Ile Phe Val
            260                 265                 270

Gly Glu Asn Ser Ser Glu Met Leu Glu Phe Val Ser Cys Phe Met Ala
        275                 280                 285

Leu Phe Met Val Gly Leu Ser Leu Phe Asn Phe Ser Ile Ala Phe Val
    290                 295                 300

Cys Met Val Ala Gly Phe Cys Ser Lys Gln Ala Val Lys Phe Lys Val
305                 310                 315                 320

Ser Trp Phe Ala Met Val Phe Ala Asn Val Gly Leu Val Met Asp Ile
                325                 330                 335

Asp Glu Ile Gly Arg Ser Val Asn Ser Ala Ala Ile Lys Met Val Gly
            340                 345                 350

Glu Ile Ala Gly Val Ala Ile Thr Val Val Trp Ile Ala Leu Phe Leu
        355                 360                 365

Met Ala Ala Arg Ala Val Tyr Val Gln Glu Ile Leu Tyr Pro Gly Lys
    370                 375                 380

Asp Glu Asn Ser Ser Thr Leu Ser His Glu Val Leu Gln Tyr Tyr Ser
385                 390                 395                 400

Ala Leu Gln Glu Thr Val Ala Thr Tyr Asn Ile Pro Ala Pro Gln Pro
                405                 410                 415

Gly Gly Asp Pro Ser Thr Ala Ala Tyr Asn Ala Asp Met Ala Pro Ile
            420                 425                 430

Asp Glu Lys Lys Asp Gly Asp Met Ser Ser Ala Tyr Arg Arg Asn Phe
        435                 440                 445

Tyr Pro Ala Val His Gly Asn Pro Thr Pro Glu Glu Ile Ile
    450                 455                 460

<210>  5
<211>  430
<212>  PRT
<213>  Actinobacillus succinogenes

<400>  5

Met Asp Glu Leu Lys Pro Ile Ile Ala Val Ala Gly Ile Ile Ile Thr
1               5                   10                  15

Val Tyr Leu Leu Ile Lys Lys His Glu Thr Arg Thr Val Leu Ile Gly
            20                  25                  30

Val Gly Leu Leu Met Ser Ile Leu Thr Leu Asn Pro Met Gly Ala Leu
        35                  40                  45

Asp Ala Phe Ala Lys Ser Met Thr Ser Gly Gly Leu Ile Met Ala Ile
    50                  55                  60

Cys Ser Ser Met Gly Phe Ala Tyr Val Met Lys Tyr Thr Gln Cys Asp
65                  70                  75                  80

Thr His Leu Val His Leu Leu Thr Lys Pro Leu Gly Gly Leu Lys Phe
                85                  90                  95

Phe Leu Ile Pro Val Ala Thr Val Ile Thr Phe Phe Ile Asn Ile Ala
            100                 105                 110

Ile Pro Ser Ala Ala Gly Cys Ser Ala Ala Val Gly Ala Thr Leu Ile
        115                 120                 125

Pro Val Leu Lys Ser Ala Gly Val Arg Pro Ala Thr Ala Gly Ala Ala
    130                 135                 140

Ile Leu Ala Gly Thr Phe Gly Ser Met Met Ser Pro Gly Ser Ser His
145                 150                 155                 160

Ser Ala Met Ile Ser Glu Met Ser Lys Leu Thr Val Thr Glu Val Asn
                165                 170                 175

Leu Ser His Ala Pro Tyr Thr Met Val Ala Gly Ala Ile Gly Ala Val
            180                 185                 190

Met Leu Thr Leu Leu Ala Leu Phe Phe Lys Asp Tyr Gly Asp Glu His
        195                 200                 205

Arg Gln Ala Tyr Leu Ser Glu Gln Lys Glu Ala Glu Glu Lys Phe Val
    210                 215                 220

Lys Val Asn Val Leu Phe Ala Leu Ala Pro Leu Val Pro Leu Val Ile
225                 230                 235                 240

Leu Val Ile Gly Gly Thr Ser Leu Gln Gln Val Ser Trp Leu Ser Trp
                245                 250                 255

Thr Lys Met Gly Val Pro Gln Ala Met Leu Ile Gly Ala Ile Tyr Gly
            260                 265                 270

Ile Leu Val Thr Arg Ile Ser Pro Val Lys Ile Thr Glu Glu Phe Phe
        275                 280                 285

Asn Gly Met Gly Asn Ser Tyr Ala Asn Val Leu Gly Ile Ile Ile Ala
    290                 295                 300

Ala Gly Val Phe Val Ala Gly Leu Lys Ser Thr Gly Ala Ile Asp Ser
305                 310                 315                 320

Ala Ile Glu Phe Leu Lys His Ser Asn Glu Phe Val Arg Trp Gly Ala
                325                 330                 335

Thr Ile Gly Pro Phe Leu Met Gly Val Ile Thr Gly Ser Gly Asp Ala
            340                 345                 350

Ala Ala Ile Ala Phe Asn Ser Ala Val Thr Pro His Ala Val Glu Leu
        355                 360                 365

Gly Tyr Thr His Val Asn Leu Gly Met Ala Ala Ala Val Ser Gly Ala
    370                 375                 380

Ile Gly Arg Thr Ala Ser Pro Ile Ala Gly Val Thr Ile Val Cys Ala
385                 390                 395                 400

Gly Leu Ala Met Val Ser Pro Val Glu Met Ile Lys Arg Thr Ala Pro
                405                 410                 415

Gly Met Val Leu Ala Ile Leu Phe Leu Ala Leu Phe Met Leu
            420                 425                 430

<210> 6
<211> 476
<212> PRT
<213> Bifidobacterium longum

<400> 6

Met Glu Leu Val Ile Met Tyr Leu Val Ala Ile Ala Ala Val Gly Ile
1               5                   10                  15

Val Gly Phe Met Leu Val Lys Lys Met Asp Ile Lys Ile Ser Leu Phe
            20                  25                  30

Leu Ile Gly Ile Val Leu Met Tyr Ile Ala Leu Leu Met Gly Lys Asp
        35                  40                  45

Ile Ser Phe Thr Asp Phe Val Ser Ser Gly Val Thr Trp Leu Asp Pro
    50                  55                  60

Phe Lys Val Val Gly Asp Gln Phe Val Ser Thr Leu Thr Ser Ala Gly
65                  70                  75                  80

Phe Ile Ile Leu Ile Leu Gly Gly Tyr Thr Gly Tyr Met Ser His Ile
                85                  90                  95

Gly Ala Asn Glu Val Thr Val Ser Val Leu Ala Lys Pro Ile Ala His
            100                 105                 110

Ile Lys Ser Pro Tyr Ile Leu Val Pro Ile Thr Phe Leu Leu Gly Asn
        115                 120                 125

Leu Leu Ser Leu Val Ile Pro Ser Ala Ser Asn Leu Ala Ile Ile Leu
    130                 135                 140

Met Ala Thr Leu Tyr Pro Val Leu Arg Gln Ala Gly Met Ser Leu Leu
145                 150                 155                 160

Ser Ala Ala Ala Val Ile Ala Thr Thr Ala Thr Ile Met Pro Thr Pro
                165                 170                 175

Leu Gly Ser Asp Asn Val Ala Ile Ala Ala Glu Leu Ala Lys Thr Asp

180                 185                 190

Met Phe Ser Gly Leu Thr Ala Ser Asp Tyr Val Phe Arg Tyr His Val
        195                 200                 205

Leu Val Ser Ile Pro Thr Leu Leu Val Met Ala Leu Ala His Tyr Phe
    210                 215                 220

Trp Gln Lys Phe Met Asp Lys Arg Ala Gly Ser Asp Leu Thr Asp Gly
225                 230                 235                 240

Asp Val Asp Leu Ala Glu Val Lys Ala Val Glu Gly Ser Ala Leu Tyr
                245                 250                 255

Arg Thr Val Tyr Ala Ile Leu Pro Leu Leu Pro Ile Ile Met Leu Leu
            260                 265                 270

Ile Val Phe Ala Ile Thr Ser Thr Thr Gly Ala Lys Ile Asp Leu Ser
        275                 280                 285

Val Glu Leu Ala Ser Ile Leu Ser Phe Val Ile Ala Ile Ile Cys Glu
    290                 295                 300

Leu Ile Arg Thr Arg Lys Gly Lys Glu Thr Leu Ala Gly Thr Glu Ser
305                 310                 315                 320

Phe Phe Lys Gly Met Gly Gly Ala Met Pro Ile Val Ala Leu Leu Val
                325                 330                 335

Ala Ala Ser Val Phe Val Val Gly Leu Lys Ser Ile Gly Leu Ile Thr
            340                 345                 350

Glu Leu Gln Asn Ala Met Thr Gly Leu Ser Gly Ser Gly Met Gly Phe
        355                 360                 365

Val Leu Pro Leu Ile Leu Val Gly Leu Thr Ala Leu Ile Val Leu Leu
    370                 375                 380

Ser Gly Ser Gly Thr Ala Leu Phe Phe Ala Met Val Pro Leu Met Val
385                 390                 395                 400

Pro Leu Ala Ala Ala Ala Gly Ile Asn Val Leu Ala Val Ser Ile Pro
                405                 410                 415

Met Gly Leu Ala Gly Asn Leu Leu Arg Ala Val Ser Pro Val Ser Ala
            420                 425                 430

Val Val Met Ile Val Ala Gly Ser Ile Lys Lys Ser Pro Ile Asp Ile
        435                 440                 445

Val Arg Arg Thr Ser Val Pro Met Ile Ala Gly Val Ile Val Met Phe
    450                 455                 460

Ile Leu Ser Met Ala Ile Phe Leu Pro Met Gly Ala
465                 470                 475

<210>  7
<211>  1198
<212>  DNA
<213>  Artificial Sequence

<220>
<223>  Synthetic gene sequence of DCT_01

<400>  7
atggaaagaa ctctatgtcc aaacaccacc ccaccagctc aagaattgac caagcacgac      60

gtcggttgga gaagaattgt cagaaacttc accccatcct ggttctccgt taccatgggt     120

actggtattg tttccatctt gttgaacact ttgccataca acgctccatg gttatactgg     180

atctctgttg tcttgttcgc tgtcaatgtc ttgttgttcg gtaccggttg tatcatctct     240

gtcttgagat acatcttgta cccagaagtc tttaccgcca tgatctctca tccagtccaa     300

tccatgttca tcggtacttt cccaatgggt ttggctacca tcatcaacat gttctgtttc     360

gtttgtgttc cagtctgggg tgaatggacc agaaactgtg cttggggttt atggattgtc     420

gatgctgtct tgtctgttgt tgttgctttg tctttgccat tcttgttgat ggctaacggt     480

cacgaaactc aattatcttc catgactgct gtctggttgt tgcctgttgt ttcttgtgtt     540

gttgctgcct cctctggtgc cattgttgct gacatcttgc caaaccctca acacgcttta     600

tggaccgtca ttgtttccta catcttatgg ggtattggtg ttccattggc tttgatggtc     660

atggttatct acttgcaaag attgactttg cacaagttgc caccaaaggc tgtcattgtc     720

tccgtctttt tgccattggg tccattgggt caaggtggtt acggtgccat gaagttgggt     780

caagctgctc aatccatctt cccacaaacc cacactttgg acgcttctgc tggtccaatg     840

ttctacactt tgggtttctt ggttgctttg atcttatggt ctttcggttt ggtctggtta     900

ttctttgctt ctgcctccat tgccagacac agaaacttcc cattcaacat tggttggtgg 960

ggtttcactt tcccattggg tgtctttgcc acttctacct gtcaaatggg tcgtgaattg 1020

ccatctgaat tcttcagagt tttgggtacc atcttgtctc tatgtgttgt tgtcttatgg 1080

atggttgttt ccatgggtac tttgaagggt gttgtttctg gtcaattgtt cttcgctcca 1140

tgtttggctg atttgaagat gaaggaagct gacggtaacg gtgaaaaaag ggcctaag 1198

<210> 8
<211> 1252
<212> DNA
<213> Artificial Sequence

<220>
<223> Synthetic gene sequence of DCT_02

<400> 8
atgaacgttg aaacttcttt gccaggttct tctggttctg acttggaaac tttccaccac 60

gaaaccaaga agcatgccaa ccacgactct ggtatttccg tcaaccatga agctgaaatt 120

ggtgttaacc acactttcga aaagccaggt ccagttggta tcagagaaag attacgtcac 180

ttcacctggg cttggtacac tttgaccatg tcctgtggtg gtttggcttt gttgattgtc 240

aaccaaccac acgacttcaa gggtttgaaa gatattgcca gagttgtcta ctgtttgaac 300

ttggctttct ttgttatcgt tacctctttg atggccatca gattcatctt gcacaagaac 360

atgtgggaat ccttgggtca cgacagagaa ggtttgtttt tcccaacttt ctggttatcc 420

attgctacca tgatcactgg tttgtacaag tgtttcggtg atgatgctaa cgaaaagttc 480

accaagtgtt tgcaagtttt gttctggatc tactgtggtt gtaccatgat cactgctgtc 540

ggtcaatact ctttcgtctt tgctacccac aaatacgaat tgcacaccat gatgccatcc 600

tggatcttgc cagctttccc agttatgttg tctggtacta tcgcctccgt catcggttct 660

ggtcaaccag cttccgatgg tattccaatt attattgctg gtatcacttt ccaaggttta 720

ggtttctcca tctccttcat gatgtacgct cactacattg gtagattgat ggaagttggt 780

ttaccatctc cagaacacag accaggtatg ttcatctgtg ttggtcctcc agctttcacc 840

gctttggctt tggtcggtat ggccaaggct ttaccagacg acttccaaat tgtcggtgac 900

cctcacgctg tcattgacgg tcgtgttatg ttgttcttgg ctgtctctgc tgccatcttc 960

ttatgggctt tgtctttctg gttcttctgt atcgctgttg ttgctgttgt cagatctcca 1020

ccaaagggtt tccatttgaa ctggtttgcc atggttttcc caaacactgg tttcaccttg 1080

gctaccatca ctttggctaa catgttcgaa tctccaggtg tcaagggtgt tgccactgct 1140

atgtccctat gtgtcatcat catgtttatt ttcgtcttgg tttctgccat cagagctgtc 1200

atcagaaagg acatcatgtg gccaggtcaa gatgaagatg tttctgaata ag 1252

<210> 9
<211> 1162
<212> DNA
<213> Artificial Sequence

<220>
<223> Synthetic gene sequence of DCT_03

<400> 9
atgaaggctt ccatggacga cattttgacc aagggtagaa acttgtccaa gggtaaggtt 60

tctttgagag atcgtatcac ttgttaccaa tggacctggt tcaccatgac catggctacc 120

ggtggtgttg ccaacgtctt gcactccatt ccatacaagt ctcaatggtt gactggtgtc 180

ggtttgtttt tcttcttctt gaacttggtc ttattcaccg tcaactgtat ctgtatcact 240

ttgagatttg tctggtccag aggttctttc ttgcactctt tgaatgacca aactgaatct 300

ttgtttgttg ctggtaccat tttcatcacc atctgtcaat acggtatccc aaacactggt 360

ccatggttat tgaaggccat ggaaatcatc ttctggattt acgttggttt gtctttctcc 420

atttctgctt tcttgtactt gctattatgg tccactaccg ttttcccaat ccacaccatg 480

accccagtct gggttttccc agcttaccca ttgttgttga ctgctccata cggtgccaac 540

ttgattgctg ctgctgtcaa atctggtcat ttgaccagag ctggtcaatc taccatcaac 600

ccaatctcca ttgctttggc ttccgttgcc gttcaaggta ccggtttctt gatctctttc 660

atgatctgtg ccgctttctt gtacagattg atgactcaaa agttgccacg tgactaccaa 720

agacctggtg tctttatttc cattggtcca ggtgctttca ccgctgctgg tttggttcaa 780

ttggccaact ctgctccaga tttcttccca actgaattct tgggtactca aggtgctgtt 840

gccatttcta tcttgagaat gttggcttac atggctggta tctggttatg gggtttgtct 900

tgttggttct tcttagtctc cgttggttct ttatggaaat acctaagacc agaaagaaag 960

tccaagttgc aattccaaat gacttggttc tctttcgttt tcccaaacac tgctttggtc 1020

actgctactg aacaattagg taaggctttc gaatctgatg gtttgaagat cttaggttgt 1080

gttttgactg gttgtatcat tgtcgtctgg attgttgttt tctggagaat gttggaatgt 1140

atctggagaa gagagttgta ag 1162

<210> 10

<211>  1390
<212>  DNA
<213>  Artificial Sequence

<220>
<223>  Synthetic gene sequence of DCT_04

<400>  10
atggttccac cattattgaa acaaagatac cacgaattgt ttgacttgag agtcagaggt      60

ccaagaattc cattgaagaa gagattggaa tgtttctcct gggcttggtt cgcctcctcc     120

atgggtactg gtggtattgc catggttatc tctttgtacc catacagatt ctacggtatg     180

aacaccatcg gtaaggtcgt tttcatcttc caattgtgtt tgttggctac ttatttcatc     240

tgtatcacct acagatttgt ccgtgttcca ggttctttgc aaaagtcctg gaccaaccca     300

gctgaaatgt tgttcatccc aacttctcta ttatccgttg ccacctccat ttcttgtttg     360

gacccatttg cttggccata cgtcggtatc tggatggttt acttcatcag agttgtctac     420

tggatcttcg tcggtgtttc tttcatcttt gtcatgggtc aattctacac tttatactcc     480

agaaacattt tcaccccaga aaccattgct ccagctttgg tcttgccaat cttcccatgt     540

atgatctgtg gtgtcattgc ttctgccatt gctgaatctc aaccagctaa gcaagctaag     600

gatatggtca ttgccggtat tgctttccaa ggtttaggtt tctgggttta catcatggtc     660

tacgctgtta acatgtacaa attgttcatc tacggtttga tgccagcttc tgacagacca     720

ggtttattca tcttcgtttc tccaccttct ttcaccggtt tgactttgtt ggatttgtct     780

ttcgctgcca gaaagaagcg tccttacatc tttgtcggtg aaaactcttc tgaaatgttg     840

gaattcgttt cctgtttcat ggctttgttc atggtcggtt tgtctttgtt caacttctcc     900

attgctttcg tttgtatggt tgccggtttc tgttccaagc aagctgtcaa attcaaggtt     960

tcctggttcg ctatggtctt tgctaacgtt ggtttggtca tggacattga cgaaatcggt    1020

cgttctgtta actctgctgc catcaagatg gttggtgaaa ttgctggtgt tgccatcact    1080

gttgtctgga tcgctttgtt cttgatggct gccagagctg tttacgtcca agaaatctta    1140

tacccaggta aggatgaaaa ctcttccact ttgtcccacg aagttttgca atactactct    1200

gctttgcaag aaactgttgc cacctacaac attccagctc ctcaaccagg tggtgatcca    1260

tctaccgctg cttacaatgc tgacatggct ccaattgacg aaaagaagga tggtgacatg    1320

tcctctgctt acagaagaaa cttctaccca gctgtccacg gtaacccaac tccagaagaa    1380

atcatttaag                                                           1390

<210>  11
<211>  1294
<212>  DNA
<213>  Artificial Sequence

<220>
<223>  Synthetic gene sequence of DCT_08

<400>  11
atggacgaat tgaagccaat cattgctgtt gctggtatca tcatcactgt ttacttgttg      60

atcaagaagc acgaaaccag aaccgtcttg attggtgttg gtctattgat gtccatcttg     120

actttgaacc caatgggtgc tttggatgct ttcgctaagt ccatgacttc tggtggtttg     180

atcatggcca tctgttcctc catgggtttc gcttacgtca tgaagtacac tcaatgtgac     240

acccacttgg ttcatttgtt gaccaagcca ttaggtggtt tgaaattctt cttgattcca     300

gttgccaccg tcatcacttt cttcatcaac attgccattc catctgctgc tggttgttct     360

gctgctgttg gtgctacttt gattccagtt ttgaaatccg ctggtgtcag accagctacc     420

gctggtgctg ccatcttggc tggtactttc ggttccatga tgtctccagg ttcttctcac     480

tctgctatga tctccgaaat gtccaagttg accgttactg aagtcaactt gtctcatgcc     540

ccatacacca tggttgctgg tgccattggt gctgtcatgt tgactttatt ggctttgttc     600

ttcaaggact acggtgatga acaccgtcaa gcctacttgt ctgaacaaaa ggaagctgaa     660

gaaaagttcg tcaaggtcaa cgtcttattc gctttggctc cattggttcc attggttatc     720

ttagtcattg gtggtacttc tctacaacaa gtctcctggt tatcctggac caagatgggt     780

gttcctcaag ctatgttgat tggtgccatc tacggtatct tggtcaccag aatctctcca     840

gtcaagatca ctgaagaatt tttcaacggt atgggtaact cttacgctaa cgttttgggt     900

atcatcattg ctgctggtgt tttcgttgct ggtttgaaat ctaccggtgc tattgactct     960

gccattgaat ttttgaagca ctccaatgaa tttgtcagat ggggtgctac cattggtcca    1020

ttcttgatgg gtgtcatcac tggttccggt gatgctgctg ccattgcttt caactctgct    1080

gtcaccccac acgctgtcga attgggttac acccacgtta acttgggtat ggctgctgct    1140

gtttctggtg ccatcggtag aactgcttct ccaattgctg gtgttaccat tgtctgtgcc    1200

ggtttggcca tggtttctcc agttgaaatg atcaagagaa ctgctccagg tatggttttg    1260

gccatcttat tcttggcttt gttcatgctt taag                                1294


<210>  12
<211>  1432
<212>  DNA

<213>  Artificial Sequence

<220>
<223>  Synthetic gene sequence of DCT_09

<400>  12
atggaattgg ttatcatgta cttggttgcc attgctgctg ttggtattgt cggtttcatg      60

ttggtcaaga agatggatat caagatctct ctattcttga ttggtattgt cttgatgtac     120

attgctctat tgatgggtaa ggacatctct ttcaccgatt ttgtctcttc tggtgtcacc     180

tggttggacc ctttcaaggt tgtcggtgac caattcgttt ccactttgac ctctgctggt     240

ttcatcatct tgattttggg tggttacact ggttacatgt cccacattgg tgctaacgaa     300

gtcactgttt ccgtcttggc caagccaatt gctcacatca aatctccata catcttggtt     360

ccaatcactt tcttgttggg taacttgttg tctttggtca ttccatctgc ttctaacttg     420

gccatcatct tgatggctac tttataccca gtcttgagac aagctggtat gtccttgttg     480

tctgctgctg ctgttatcgc taccactgct accatcatgc caactccatt aggttccgac     540

aacgttgcca ttgctgctga attggccaag accgatatgt tctccggttt gaccgcttct     600

gactacgttt tccgttacca cgtcttggtt tccattccaa ctttattggt tatggctttg     660

gctcactact tctggcaaaa gttcatggac aagagagctg gttctgattt gactgacggt     720

gatgttgact tggctgaagt caaggctgtt gaaggttctg ctttgtacag aaccgtctac     780

gctatcttac cattactacc aatcatcatg ttgttgattg tctttgccat cacctccacc     840

accggtgcca agattgactt gtctgttgaa ttggcttcca tcttatcttt cgtcattgcc     900

atcatctgtg aattgatcag aaccagaaag ggtaaggaaa ctttggccgg tactgaatct     960

ttcttcaagg gtatgggtgg tgccatgcca atcgttgctt tgttggttgc tgcttccgtt    1020

ttcgttgttg gtttgaaatc catcggtttg atcactgaat tgcaaaacgc tatgaccggt    1080

ttgtccggtt ctggtatggg tttcgtctta ccattgatct tagtcggttt gaccgctttg    1140

attgtcttgt tgtccggttc tggtactgct ttgttctttg ccatggttcc tttgatggtt    1200

ccattagctg ctgctgctgg tatcaacgtc ttggccgttt ccattccaat gggtttggct    1260

ggtaacttgt tgagagctgt ctctccagtt tctgctgtcg ttatgattgt tgctggttct    1320

atcaagaaat ctccaattga tattgtcaga agaacttctg ttccaatgat tgctggtgtc    1380

atcgttatgt tcatcttatc catggccatc ttcttgccaa tgggggcata ag            1432


<210>  13
<211>  1139

<212>  PRT
<213>  Artificial Sequence

<220>
<223>  Glycosomal Trypanosoma brucei fumarate reductase (FRDg) amino
       acid sequence lacking 3 aa C-terminal targeting signal.

<400>  13

Met Val Asp Gly Arg Ser Ser Ala Ser Ile Val Ala Val Asp Pro Glu
1               5                   10                  15

Arg Ala Ala Arg Glu Arg Asp Ala Ala Ala Arg Ala Leu Leu Gln Asp
            20                  25                  30

Ser Pro Leu His Thr Thr Met Gln Tyr Ala Thr Ser Gly Leu Glu Leu
        35                  40                  45

Thr Val Pro Tyr Ala Leu Lys Val Val Ala Ser Ala Asp Thr Phe Asp
    50                  55                  60

Arg Ala Lys Glu Val Ala Asp Glu Val Leu Arg Cys Ala Trp Gln Leu
65                  70                  75                  80

Ala Asp Thr Val Leu Asn Ser Phe Asn Pro Asn Ser Glu Val Ser Leu
                85                  90                  95

Val Gly Arg Leu Pro Val Gly Gln Lys His Gln Met Ser Ala Pro Leu
            100                 105                 110

Lys Arg Val Met Ala Cys Cys Gln Arg Val Tyr Asn Ser Ser Ala Gly
        115                 120                 125

Cys Phe Asp Pro Ser Thr Ala Pro Val Ala Lys Ala Leu Arg Glu Ile
    130                 135                 140

Ala Leu Gly Lys Glu Arg Asn Asn Ala Cys Leu Glu Ala Leu Thr Gln
145                 150                 155                 160

Ala Cys Thr Leu Pro Asn Ser Phe Val Ile Asp Phe Glu Ala Gly Thr
                165                 170                 175

Ile Ser Arg Lys His Glu His Ala Ser Leu Asp Leu Gly Gly Val Ser
            180                 185                 190

Lys Gly Tyr Ile Val Asp Tyr Val Ile Asp Asn Ile Asn Ala Ala Gly
        195                 200                 205

Phe Gln Asn Val Phe Phe Asp Trp Gly Gly Asp Cys Arg Ala Ser Gly
    210                 215                 220

Met Asn Ala Arg Asn Thr Pro Trp Val Val Gly Ile Thr Arg Pro Pro
225                 230                 235                 240

Ser Leu Asp Met Leu Pro Asn Pro Pro Lys Glu Ala Ser Tyr Ile Ser
                245                 250                 255

Val Ile Ser Leu Asp Asn Glu Ala Leu Ala Thr Ser Gly Asp Tyr Glu
            260                 265                 270

Asn Leu Ile Tyr Thr Ala Asp Asp Lys Pro Leu Thr Cys Thr Tyr Asp
        275                 280                 285

Trp Lys Gly Lys Glu Leu Met Lys Pro Ser Gln Ser Asn Ile Ala Gln
    290                 295                 300

Val Ser Val Lys Cys Tyr Ser Ala Met Tyr Ala Asp Ala Leu Ala Thr
305                 310                 315                 320

Ala Cys Phe Ile Lys Arg Asp Pro Ala Lys Val Arg Gln Leu Leu Asp
                325                 330                 335

Gly Trp Arg Tyr Val Arg Asp Thr Val Arg Asp Tyr Arg Val Tyr Val
            340                 345                 350

Arg Glu Asn Glu Arg Val Ala Lys Met Phe Glu Ile Ala Thr Glu Asp
        355                 360                 365

Ala Glu Met Arg Lys Arg Arg Ile Ser Asn Thr Leu Pro Ala Arg Val
    370                 375                 380

Ile Val Val Gly Gly Gly Leu Ala Gly Leu Ser Ala Ala Ile Glu Ala
385                 390                 395                 400

Ala Gly Cys Gly Ala Gln Val Val Leu Met Glu Lys Glu Ala Lys Leu
                405                 410                 415

Gly Gly Asn Ser Ala Lys Ala Thr Ser Gly Ile Asn Gly Trp Gly Thr

            420                 425                 430

Arg Ala Gln Ala Lys Ala Ser Ile Val Asp Gly Gly Lys Tyr Phe Glu
        435                 440                 445

Arg Asp Thr Tyr Lys Ser Gly Ile Gly Gly Asn Thr Asp Pro Ala Leu
    450                 455                 460

Val Lys Thr Leu Ser Met Lys Ser Ala Asp Ala Ile Gly Trp Leu Thr
465                 470                 475                 480

Ser Leu Gly Val Pro Leu Thr Val Leu Ser Gln Leu Gly Gly His Ser
                485                 490                 495

Arg Lys Arg Thr His Arg Ala Pro Asp Lys Lys Asp Gly Thr Pro Leu
            500                 505                 510

Pro Ile Gly Phe Thr Ile Met Lys Thr Leu Glu Asp His Val Arg Gly
        515                 520                 525

Asn Leu Ser Gly Arg Ile Thr Ile Met Glu Asn Cys Ser Val Thr Ser
    530                 535                 540

Leu Leu Ser Glu Thr Lys Glu Arg Pro Asp Gly Thr Lys Gln Ile Arg
545                 550                 555                 560

Val Thr Gly Val Glu Phe Thr Gln Ala Gly Ser Gly Lys Thr Thr Ile
                565                 570                 575

Leu Ala Asp Ala Val Ile Leu Ala Thr Gly Gly Phe Ser Asn Asp Lys
            580                 585                 590

Thr Ala Asp Ser Leu Leu Arg Glu His Ala Pro His Leu Val Asn Phe
        595                 600                 605

Pro Thr Thr Asn Gly Pro Trp Ala Thr Gly Asp Gly Val Lys Leu Ala
    610                 615                 620

Gln Arg Leu Gly Ala Gln Leu Val Asp Met Asp Lys Val Gln Leu His
625                 630                 635                 640

Pro Thr Gly Leu Ile Asn Pro Lys Asp Pro Ala Asn Pro Thr Lys Phe
                645                 650                 655

Leu Gly Pro Glu Ala Leu Arg Gly Ser Gly Gly Val Leu Leu Asn Lys
            660                 665                 670

Gln Gly Lys Arg Phe Val Asn Glu Leu Asp Leu Arg Ser Val Val Ser
        675                 680                 685

Lys Ala Ile Met Glu Gln Gly Ala Glu Tyr Pro Gly Ser Gly Gly Ser
    690                 695                 700

Met Phe Ala Tyr Cys Val Leu Asn Ala Ala Ala Gln Lys Leu Phe Gly
705                 710                 715                 720

Val Ser Ser His Glu Phe Tyr Trp Lys Lys Met Gly Leu Phe Val Lys
                725                 730                 735

Ala Asp Thr Met Arg Asp Leu Ala Ala Leu Ile Gly Cys Pro Val Glu
            740                 745                 750

Ser Val Gln Gln Thr Leu Glu Glu Tyr Glu Arg Leu Ser Ile Ser Gln
        755                 760                 765

Arg Ser Cys Pro Ile Thr Arg Lys Ser Val Tyr Pro Cys Val Leu Gly
    770                 775                 780

Thr Lys Gly Pro Tyr Tyr Val Ala Phe Val Thr Pro Ser Ile His Tyr
785                 790                 795                 800

Thr Met Gly Gly Cys Leu Ile Ser Pro Ser Ala Glu Ile Gln Met Lys
                805                 810                 815

Asn Thr Ser Ser Arg Ala Pro Leu Ser His Ser Asn Pro Ile Leu Gly
            820                 825                 830

Leu Phe Gly Ala Gly Glu Val Thr Gly Gly Val His Gly Gly Asn Arg
        835                 840                 845

Leu Gly Gly Asn Ser Leu Leu Glu Cys Val Val Phe Gly Arg Ile Ala
    850                 855                 860

Gly Asp Arg Ala Ser Thr Ile Leu Gln Arg Lys Ser Ser Ala Leu Ser
865                 870                 875                 880

Phe Lys Val Trp Thr Thr Val Val Leu Arg Glu Val Arg Glu Gly Gly
                885                 890                 895

Val Tyr Gly Ala Gly Ser Arg Val Leu Arg Phe Asn Leu Pro Gly Ala
            900                 905                 910

Leu Gln Arg Ser Gly Leu Ser Leu Gly Gln Phe Ile Ala Ile Arg Gly
        915                 920                 925

Asp Trp Asp Gly Gln Gln Leu Ile Gly Tyr Tyr Ser Pro Ile Thr Leu
    930                 935                 940

Pro Asp Asp Leu Gly Met Ile Asp Ile Leu Ala Arg Ser Asp Lys Gly
945                 950                 955                 960

Thr Leu Arg Glu Trp Ile Ser Ala Leu Glu Pro Gly Asp Ala Val Glu
                965                 970                 975

Met Lys Ala Cys Gly Gly Leu Val Ile Glu Arg Arg Leu Ser Asp Lys
            980                 985                 990

His Phe Val Phe Met Gly His Ile  Ile Asn Lys Leu Cys  Leu Ile Ala
        995                 1000                 1005

Gly Gly  Thr Gly Val Ala Pro  Met Leu Gln Ile Ile  Lys Ala Ala
    1010                 1015                 1020

Phe Met  Lys Pro Phe Ile Asp  Thr Leu Glu Ser Val  His Leu Ile
    1025                 1030                 1035

Tyr Ala  Ala Glu Asp Val Thr  Glu Leu Thr Tyr Arg  Glu Val Leu
    1040                 1045                 1050

Glu Glu  Arg Arg Arg Glu Ser  Arg Gly Lys Phe Lys  Lys Thr Phe
    1055                 1060                 1065

Val Leu  Asn Arg Pro Pro Pro  Leu Trp Thr Asp Gly  Val Gly Phe
    1070                 1075                 1080

Ile Asp  Arg Gly Ile Leu Thr  Asn His Val Gln Pro  Pro Ser Asp
    1085                 1090                 1095

Asn Leu  Leu Val Ala Ile Cys  Gly Pro Pro Val Met  Gln Arg Ile
    1100                 1105                 1110

Val Lys  Ala Thr Leu Lys Thr  Leu Gly Tyr Asn Met  Asn Leu Val
    1115                 1120                 1125

Arg Thr  Val Asp Glu Thr Glu  Pro Ser Gly Ser
    1130                 1135

<210>  14
<211>  472
<212>  PRT
<213>  Artificial Sequence

<220>
<223>  Rhizopus oryzae fumarase amino acid sequence, lacking the first
       23 N-terminal amino acids.

<400>  14

Met Ser Ser Ala Ser Ala Ala Leu Gln Lys Phe Arg Ala Glu Arg Asp
1               5                   10                  15

Thr Phe Gly Asp Leu Gln Val Pro Ala Asp Arg Tyr Trp Gly Ala Gln
            20                  25                  30

Thr Gln Arg Ser Leu Gln Asn Phe Asp Ile Gly Gly Pro Thr Glu Arg
        35                  40                  45

Met Pro Glu Pro Leu Ile Arg Ala Phe Gly Val Leu Lys Lys Ala Ala
    50                  55                  60

Ala Thr Val Asn Met Thr Tyr Gly Leu Asp Pro Lys Val Gly Glu Ala
65                  70                  75                  80

Ile Gln Lys Ala Ala Asp Glu Val Ile Asp Gly Ser Leu Ile Asp His
                85                  90                  95

Phe Pro Leu Val Val Trp Gln Thr Gly Ser Gly Thr Gln Thr Lys Met
            100                 105                 110

Asn Val Asn Glu Val Ile Ser Asn Arg Ala Ile Glu Leu Leu Gly Gly
        115                 120                 125

Glu Leu Gly Ser Lys Ala Pro Val His Pro Asn Asp His Val Asn Met
    130                 135                 140

Ser Gln Ser Ser Asn Asp Thr Phe Pro Thr Ala Met His Val Ala Ala
145                 150                 155                 160

Val Val Glu Ile His Gly Arg Leu Ile Pro Ala Leu Thr Thr Leu Arg
                165                 170                 175

Asp Ala Leu Gln Ala Lys Ser Ala Glu Phe Glu His Ile Ile Lys Ile
            180                 185                 190

Gly Arg Thr His Leu Gln Asp Ala Thr Pro Leu Thr Leu Gly Gln Glu
        195                 200                 205

Phe Ser Gly Tyr Thr Gln Gln Leu Thr Tyr Gly Ile Ala Arg Val Gln
    210                 215                 220

Gly Thr Leu Glu Arg Leu Tyr Asn Leu Ala Gln Gly Gly Thr Ala Val
225                 230                 235                 240

Gly Thr Gly Leu Asn Thr Arg Lys Gly Phe Asp Ala Lys Val Ala Glu
                245                 250                 255

Ala Ile Ala Ser Ile Thr Gly Leu Pro Phe Lys Thr Ala Pro Asn Lys
            260                 265                 270

Phe Glu Ala Leu Ala Ala His Asp Ala Leu Val Glu Ala His Gly Ala
        275                 280                 285

Leu Asn Thr Val Ala Cys Ser Leu Met Lys Ile Ala Asn Asp Ile Arg
    290                 295                 300

Tyr Leu Gly Ser Gly Pro Arg Cys Gly Leu Gly Glu Leu Ser Leu Pro
305                 310                 315                 320

Glu Asn Glu Pro Gly Ser Ser Ile Met Pro Gly Lys Val Asn Pro Thr
                325                 330                 335

Gln Cys Glu Ala Met Thr Met Val Cys Ala Gln Val Met Gly Asn Asn
            340                 345                 350

Thr Ala Ile Ser Val Ala Gly Ser Asn Gly Gln Phe Glu Leu Asn Val
        355                 360                 365

Phe Lys Pro Val Met Ile Lys Asn Leu Ile Gln Ser Ile Arg Leu Ile
    370                 375                 380

Ser Asp Ala Ser Ile Ser Phe Thr Lys Asn Cys Val Val Gly Ile Glu
385                 390                 395                 400

Ala Asn Glu Lys Lys Ile Ser Ser Ile Met Asn Glu Ser Leu Met Leu
                405                 410                 415

Val Thr Ala Leu Asn Pro His Ile Gly Tyr Asp Lys Ala Ala Lys Cys
            420                 425                 430

Ala Lys Lys Ala His Lys Glu Gly Thr Thr Leu Lys Glu Ala Ala Leu
        435                 440                 445

Ser Leu Gly Tyr Leu Thr Ser Glu Glu Phe Asp Gln Trp Val Arg Pro
    450                 455                 460

Glu Asp Met Ile Ser Ala Lys Asp
465                 470

<210>  15
<211>  538
<212>  PRT
<213>  Artificial Sequence

<220>
<223>  Actinobacillus succinogenes phosphoenolpyruvate carboxykinase
       amino acid sequence, with EGY to DAF modification at pos 120 -
       122.

<400>  15

Met Thr Asp Leu Asn Lys Leu Val Lys Glu Leu Asn Asp Leu Gly Leu
1               5                   10                  15

Thr Asp Val Lys Glu Ile Val Tyr Asn Pro Ser Tyr Glu Gln Leu Phe
            20                  25                  30

Glu Glu Glu Thr Lys Pro Gly Leu Glu Gly Phe Asp Lys Gly Thr Leu
        35                  40                  45

Thr Thr Leu Gly Ala Val Ala Val Asp Thr Gly Ile Phe Thr Gly Arg
    50                  55                  60

Ser Pro Lys Asp Lys Tyr Ile Val Cys Asp Glu Thr Thr Lys Asp Thr
65                  70                  75                  80

Val Trp Trp Asn Ser Glu Ala Ala Lys Asn Asp Asn Lys Pro Met Thr
                85                  90                  95

Gln Glu Thr Trp Lys Ser Leu Arg Glu Leu Val Ala Lys Gln Leu Ser
            100                 105                 110

Gly Lys Arg Leu Phe Val Val Asp Ala Phe Cys Gly Ala Ser Glu Lys
        115                 120                 125

His Arg Ile Gly Val Arg Met Val Thr Glu Val Ala Trp Gln Ala His
    130                 135                 140

Phe Val Lys Asn Met Phe Ile Arg Pro Thr Asp Glu Glu Leu Lys Asn
145                 150                 155                 160

Phe Lys Ala Asp Phe Thr Val Leu Asn Gly Ala Lys Cys Thr Asn Pro
                165                 170                 175

Asn Trp Lys Glu Gln Gly Leu Asn Ser Glu Asn Phe Val Ala Phe Asn
            180                 185                 190

Ile Thr Glu Gly Ile Gln Leu Ile Gly Gly Thr Trp Tyr Gly Gly Glu
        195                 200                 205

Met Lys Lys Gly Met Phe Ser Met Met Asn Tyr Phe Leu Pro Leu Lys
    210                 215                 220

Gly Val Ala Ser Met His Cys Ser Ala Asn Val Gly Lys Asp Gly Asp
225                 230                 235                 240

Val Ala Ile Phe Phe Gly Leu Ser Gly Thr Gly Lys Thr Thr Leu Ser
                245                 250                 255

Thr Asp Pro Lys Arg Gln Leu Ile Gly Asp Asp Glu His Gly Trp Asp
            260                 265                 270

Glu Ser Gly Val Phe Asn Phe Glu Gly Gly Cys Tyr Ala Lys Thr Ile
        275                 280                 285

Asn Leu Ser Gln Glu Asn Glu Pro Asp Ile Tyr Gly Ala Ile Arg Arg

290                 295                 300

Asp Ala Leu Leu Glu Asn Val Val Val Arg Ala Asp Gly Ser Val Asp
305                 310                 315                 320

Phe Asp Asp Gly Ser Lys Thr Glu Asn Thr Arg Val Ser Tyr Pro Ile
                325                 330                 335

Tyr His Ile Asp Asn Ile Val Arg Pro Val Ser Lys Ala Gly His Ala
            340                 345                 350

Thr Lys Val Ile Phe Leu Thr Ala Asp Ala Phe Gly Val Leu Pro Pro
        355                 360                 365

Val Ser Lys Leu Thr Pro Glu Gln Thr Glu Tyr Tyr Phe Leu Ser Gly
    370                 375                 380

Phe Thr Ala Lys Leu Ala Gly Thr Glu Arg Gly Val Thr Glu Pro Thr
385                 390                 395                 400

Pro Thr Phe Ser Ala Cys Phe Gly Ala Ala Phe Leu Ser Leu His Pro
                405                 410                 415

Ile Gln Tyr Ala Asp Val Leu Val Glu Arg Met Lys Ala Ser Gly Ala
            420                 425                 430

Glu Ala Tyr Leu Val Asn Thr Gly Trp Asn Gly Thr Gly Lys Arg Ile
        435                 440                 445

Ser Ile Lys Asp Thr Arg Gly Ile Ile Asp Ala Ile Leu Asp Gly Ser
    450                 455                 460

Ile Glu Lys Ala Glu Met Gly Glu Leu Pro Ile Phe Asn Leu Ala Ile
465                 470                 475                 480

Pro Lys Ala Leu Pro Gly Val Asp Pro Ala Ile Leu Asp Pro Arg Asp
                485                 490                 495

Thr Tyr Ala Asp Lys Ala Gln Trp Gln Val Lys Ala Glu Asp Leu Ala
            500                 505                 510

Asn Arg Phe Val Lys Asn Phe Val Lys Tyr Thr Ala Asn Pro Glu Ala
        515                 520                 525

Ala Lys Leu Val Gly Ala Gly Pro Lys Ala
    530                 535

<210>  16
<211>  340
<212>  PRT
<213>  Artificial Sequence

<220>
<223>  Saccharomyces cerevisiae peroxisomal malate dehydrogenase (Mdh3)
       amino acid sequence, lacking the 3 C-terminal peroxisomal
       targeting sequence (SKL).

<400>  16

Met Val Lys Val Ala Ile Leu Gly Ala Ser Gly Gly Val Gly Gln Pro
1               5                   10                  15

Leu Ser Leu Leu Leu Lys Leu Ser Pro Tyr Val Ser Glu Leu Ala Leu
            20                  25                  30

Tyr Asp Ile Arg Ala Ala Glu Gly Ile Gly Lys Asp Leu Ser His Ile
        35                  40                  45

Asn Thr Asn Ser Ser Cys Val Gly Tyr Asp Lys Asp Ser Ile Glu Asn
    50                  55                  60

Thr Leu Ser Asn Ala Gln Val Val Leu Ile Pro Ala Gly Val Pro Arg
65                  70                  75                  80

Lys Pro Gly Leu Thr Arg Asp Asp Leu Phe Lys Met Asn Ala Gly Ile
                85                  90                  95

Val Lys Ser Leu Val Thr Ala Val Gly Lys Phe Ala Pro Asn Ala Arg
            100                 105                 110

Ile Leu Val Ile Ser Asn Pro Val Asn Ser Leu Val Pro Ile Ala Val
        115                 120                 125

Glu Thr Leu Lys Lys Met Gly Lys Phe Lys Pro Gly Asn Val Met Gly
    130                 135                 140

Val Thr Asn Leu Asp Leu Val Arg Ala Glu Thr Phe Leu Val Asp Tyr
145                 150                 155                 160

Leu Met Leu Lys Asn Pro Lys Ile Gly Gln Glu Gln Asp Lys Thr Thr
                165                 170                 175

Met His Arg Lys Val Thr Val Ile Gly Gly His Ser Gly Glu Thr Ile
            180                 185                 190

Ile Pro Ile Ile Thr Asp Lys Ser Leu Val Phe Gln Leu Asp Lys Gln
        195                 200                 205

Tyr Glu His Phe Ile His Arg Val Gln Phe Gly Gly Asp Glu Ile Val
    210                 215                 220

Lys Ala Lys Gln Gly Ala Gly Ser Ala Thr Leu Ser Met Ala Phe Ala
225                 230                 235                 240

Gly Ala Lys Phe Ala Glu Glu Val Leu Arg Ser Phe His Asn Glu Lys
                245                 250                 255

Pro Glu Thr Glu Ser Leu Ser Ala Phe Val Tyr Leu Pro Gly Leu Lys
            260                 265                 270

Asn Gly Lys Lys Ala Gln Gln Leu Val Gly Asp Asn Ser Ile Glu Tyr
        275                 280                 285

Phe Ser Leu Pro Ile Val Leu Arg Asn Gly Ser Val Val Ser Ile Asp
    290                 295                 300

Thr Ser Val Leu Glu Lys Leu Ser Pro Arg Glu Glu Gln Leu Val Asn
305                 310                 315                 320

Thr Ala Val Lys Glu Leu Arg Lys Asn Ile Glu Lys Gly Lys Ser Phe
                325                 330                 335

Ile Leu Asp Ser
            340

<210>  17
<211>  1180
<212>  PRT
<213>  Saccharomyces cerevisiae

<400>  17

Met Ser Ser Ser Lys Lys Leu Ala Gly Leu Arg Asp Asn Phe Ser Leu

1               5                   10                  15

Leu Gly Glu Lys Asn Lys Ile Leu Val Ala Asn Arg Gly Glu Ile Pro
            20                  25                  30

Ile Arg Ile Phe Arg Ser Ala His Glu Leu Ser Met Arg Thr Ile Ala
        35                  40                  45

Ile Tyr Ser His Glu Asp Arg Leu Ser Met His Arg Leu Lys Ala Asp
    50                  55                  60

Glu Ala Tyr Val Ile Gly Glu Glu Gly Gln Tyr Thr Pro Val Gly Ala
65                  70                  75                  80

Tyr Leu Ala Met Asp Glu Ile Ile Glu Ile Ala Lys Lys His Lys Val
                85                  90                  95

Asp Phe Ile His Pro Gly Tyr Gly Phe Leu Ser Glu Asn Ser Glu Phe
            100                 105                 110

Ala Asp Lys Val Val Lys Ala Gly Ile Thr Trp Ile Gly Pro Pro Ala
        115                 120                 125

Glu Val Ile Asp Ser Val Gly Asp Lys Val Ser Ala Arg His Leu Ala
    130                 135                 140

Ala Arg Ala Asn Val Pro Thr Val Pro Gly Thr Pro Gly Pro Ile Glu
145                 150                 155                 160

Thr Val Gln Glu Ala Leu Asp Phe Val Asn Glu Tyr Gly Tyr Pro Val
                165                 170                 175

Ile Ile Lys Ala Ala Phe Gly Gly Gly Gly Arg Gly Met Arg Val Val
            180                 185                 190

Arg Glu Gly Asp Asp Val Ala Asp Ala Phe Gln Arg Ala Thr Ser Glu
        195                 200                 205

Ala Arg Thr Ala Phe Gly Asn Gly Thr Cys Phe Val Glu Arg Phe Leu
    210                 215                 220

Asp Lys Pro Lys His Ile Glu Val Gln Leu Leu Ala Asp Asn His Gly
225                 230                 235                 240

Asn Val Val His Leu Phe Glu Arg Asp Cys Ser Val Gln Arg Arg His
                245                 250                 255

Gln Lys Val Val Glu Val Ala Pro Ala Lys Thr Leu Pro Arg Glu Val
            260                 265                 270

Arg Asp Ala Ile Leu Thr Asp Ala Val Lys Leu Ala Lys Val Cys Gly
        275                 280                 285

Tyr Arg Asn Ala Gly Thr Ala Glu Phe Leu Val Asp Asn Gln Asn Arg
    290                 295                 300

His Tyr Phe Ile Glu Ile Asn Pro Arg Ile Gln Val Glu His Thr Ile
305                 310                 315                 320

Thr Glu Glu Ile Thr Gly Ile Asp Ile Val Ser Ala Gln Ile Gln Ile
                325                 330                 335

Ala Ala Gly Ala Thr Leu Thr Gln Leu Gly Leu Leu Gln Asp Lys Ile
            340                 345                 350

Thr Thr Arg Gly Phe Ser Ile Gln Cys Arg Ile Thr Thr Glu Asp Pro
        355                 360                 365

Ser Lys Asn Phe Gln Pro Asp Thr Gly Arg Leu Glu Val Tyr Arg Ser
    370                 375                 380

Ala Gly Gly Asn Gly Val Arg Leu Asp Gly Gly Asn Ala Tyr Ala Gly
385                 390                 395                 400

Ala Thr Ile Ser Pro His Tyr Asp Ser Met Leu Val Lys Cys Ser Cys
                405                 410                 415

Ser Gly Ser Thr Tyr Glu Ile Val Arg Arg Lys Met Ile Arg Ala Leu
            420                 425                 430

Ile Glu Phe Arg Ile Arg Gly Val Lys Thr Asn Ile Pro Phe Leu Leu
        435                 440                 445

Thr Leu Leu Thr Asn Pro Val Phe Ile Glu Gly Thr Tyr Trp Thr Thr
    450                 455                 460

Phe Ile Asp Asp Thr Pro Gln Leu Phe Gln Met Val Ser Ser Gln Asn
465                 470                 475                 480

Arg Ala Gln Lys Leu Leu His Tyr Leu Ala Asp Leu Ala Val Asn Gly
                485                 490                 495

Ser Ser Ile Lys Gly Gln Ile Gly Leu Pro Lys Leu Lys Ser Asn Pro
            500                 505                 510

Ser Val Pro His Leu His Asp Ala Gln Gly Asn Val Ile Asn Val Thr
        515                 520                 525

Lys Ser Ala Pro Pro Ser Gly Trp Arg Gln Val Leu Leu Glu Lys Gly
    530                 535                 540

Pro Ser Glu Phe Ala Lys Gln Val Arg Gln Phe Asn Gly Thr Leu Leu
545                 550                 555                 560

Met Asp Thr Thr Trp Arg Asp Ala His Gln Ser Leu Leu Ala Thr Arg
                565                 570                 575

Val Arg Thr His Asp Leu Ala Thr Ile Ala Pro Thr Thr Ala His Ala
            580                 585                 590

Leu Ala Gly Ala Phe Ala Leu Glu Cys Trp Gly Gly Ala Thr Phe Asp
        595                 600                 605

Val Ala Met Arg Phe Leu His Glu Asp Pro Trp Glu Arg Leu Arg Lys
    610                 615                 620

Leu Arg Ser Leu Val Pro Asn Ile Pro Phe Gln Met Leu Leu Arg Gly
625                 630                 635                 640

Ala Asn Gly Val Ala Tyr Ser Ser Leu Pro Asp Asn Ala Ile Asp His
                645                 650                 655

Phe Val Lys Gln Ala Lys Asp Asn Gly Val Asp Ile Phe Arg Val Phe
            660                 665                 670

Asp Ala Leu Asn Asp Leu Glu Gln Leu Lys Val Gly Val Asn Ala Val
        675                 680                 685

Lys Lys Ala Gly Gly Val Val Glu Ala Thr Val Cys Tyr Ser Gly Asp
    690                 695                 700

Met Leu Gln Pro Gly Lys Lys Tyr Asn Leu Asp Tyr Tyr Leu Glu Val
705                 710                 715                 720

Val Glu Lys Ile Val Gln Met Gly Thr His Ile Leu Gly Ile Lys Asp
                725                 730                 735

Met Ala Gly Thr Met Lys Pro Ala Ala Ala Lys Leu Leu Ile Gly Ser
            740                 745                 750

Leu Arg Thr Arg Tyr Pro Asp Leu Pro Ile His Val His Ser His Asp
        755                 760                 765

Ser Ala Gly Thr Ala Val Ala Ser Met Thr Ala Cys Ala Leu Ala Gly
    770                 775                 780

Ala Asp Val Val Asp Val Ala Ile Asn Ser Met Ser Gly Leu Thr Ser
785                 790                 795                 800

Gln Pro Ser Ile Asn Ala Leu Leu Ala Ser Leu Glu Gly Asn Ile Asp
                805                 810                 815

Thr Gly Ile Asn Val Glu His Val Arg Glu Leu Asp Ala Tyr Trp Ala
            820                 825                 830

Glu Met Arg Leu Leu Tyr Ser Cys Phe Glu Ala Asp Leu Lys Gly Pro
        835                 840                 845

Asp Pro Glu Val Tyr Gln His Glu Ile Pro Gly Gly Gln Leu Thr Asn
    850                 855                 860

Leu Leu Phe Gln Ala Gln Gln Leu Gly Leu Gly Glu Gln Trp Ala Glu
865                 870                 875                 880

Thr Lys Arg Ala Tyr Arg Glu Ala Asn Tyr Leu Leu Gly Asp Ile Val
                885                 890                 895

Lys Val Thr Pro Thr Ser Lys Val Val Gly Asp Leu Ala Gln Phe Met
            900                 905                 910

Val Ser Asn Lys Leu Thr Ser Asp Asp Ile Arg Arg Leu Ala Asn Ser

915                 920                 925

Leu Asp Phe Pro Asp Ser Val Met Asp Phe Phe Glu Gly Leu Ile Gly
    930                 935                 940

Gln Pro Tyr Gly Gly Phe Pro Glu Pro Leu Arg Ser Asp Val Leu Arg
945                 950                 955                 960

Asn Lys Arg Arg Lys Leu Thr Cys Arg Pro Gly Leu Glu Leu Glu Pro
                965                 970                 975

Phe Asp Leu Glu Lys Ile Arg Glu Asp Leu Gln Asn Arg Phe Gly Asp
            980                 985                 990

Ile Asp Glu Cys Asp Val Ala Ser  Tyr Asn Met Tyr Pro  Arg Val Tyr
        995                 1000                1005

Glu Asp  Phe Gln Lys Ile Arg  Glu Thr Tyr Gly Asp  Leu Ser Val
    1010                1015                1020

Leu Pro  Thr Lys Asn Phe Leu  Ala Pro Ala Glu Pro  Asp Glu Glu
    1025                1030                1035

Ile Glu  Val Thr Ile Glu Gln  Gly Lys Thr Leu Ile  Ile Lys Leu
    1040                1045                1050

Gln Ala  Val Gly Asp Leu Asn  Lys Lys Thr Gly Gln  Arg Glu Val
    1055                1060                1065

Tyr Phe  Glu Leu Asn Gly Glu  Leu Arg Lys Ile Arg  Val Ala Asp
    1070                1075                1080

Lys Ser  Gln Asn Ile Gln Ser  Val Ala Lys Pro Lys  Ala Asp Val
    1085                1090                1095

His Asp  Thr His Gln Ile Gly  Ala Pro Met Ala Gly  Val Ile Ile
    1100                1105                1110

Glu Val  Lys Val His Lys Gly  Ser Leu Val Lys Lys  Gly Glu Ser
    1115                1120                1125

Ile Ala  Val Leu Ser Ala Met  Lys Met Glu Met Val  Val Ser Ser
    1130                1135                1140

Pro Ala Asp Gly Gln Val Lys Asp Val Phe Ile Lys Asp Gly Glu
    1145                1150                1155

Ser Val Asp Ala Ser Asp Leu Leu Val Val Leu Glu Glu Glu Thr
    1160                1165                1170

Leu Pro Pro Ser Gln Lys Lys
    1175                1180

<210> 18
<211> 542
<212> PRT
<213> Kluyveromyces lactis

<400> 18

Met Val Ser Val Lys Ala Ser Ala Ala Glu Lys Lys Glu Phe Leu Gln
1               5                   10                  15

Ser Gln Ile Asp Glu Ile Glu Lys Trp Trp Ser Glu Pro Arg Trp Lys
            20                  25                  30

Asp Thr Lys Arg Ile Tyr Ser Ala Tyr Glu Ile Ala Lys Arg Arg Gly
        35                  40                  45

Ser Val Lys Pro Asn Thr Phe Pro Ser Thr Val Met Ser Gln Lys Leu
    50                  55                  60

Phe Lys Ile Leu Gly Glu His Ala Lys Asn Gly Thr Val Ser Lys Thr
65                  70                  75                  80

Phe Gly Ala Leu Asp Pro Val Gln Val Thr Gln Met Ser Lys Tyr Leu
                85                  90                  95

Asp Thr Ile Tyr Val Ser Gly Trp Gln Cys Ser Ser Thr Ala Ser Thr
            100                 105                 110

Ser Asn Glu Pro Gly Pro Asp Leu Ala Asp Tyr Pro Met Asp Thr Val
        115                 120                 125

Pro Asn Lys Val Glu His Leu Phe Lys Ala Gln Gln Phe His Asp Arg
    130                 135                 140

Lys Gln Trp Glu Arg Ile Cys Asp Gly Thr Ile Glu Glu Ser Glu Ile
145                 150                 155                 160

Ile Asp Tyr Leu Thr Pro Ile Val Ala Asp Gly Asp Ala Gly His Gly
                165                 170                 175

Gly Leu Thr Ala Val Phe Lys Leu Thr Lys Met Phe Ile Glu Arg Gly
            180                 185                 190

Ala Ala Gly Ile His Ile Glu Asp Gln Thr Ser Thr Asn Lys Lys Cys
        195                 200                 205

Gly His Met Ala Gly Arg Cys Val Ile Pro Val Gln Glu His Ile Asn
    210                 215                 220

Arg Leu Ile Thr Cys Arg Met Ala Ala Asp Val Leu Gly Ser Asp Leu
225                 230                 235                 240

Ile Leu Val Ala Arg Thr Asp Ser Glu Ala Ala Thr Leu Leu Ser Ser
                245                 250                 255

Thr Ala Asp Ser Arg Asp His Tyr Phe Ile Leu Gly Ala Ser Asn Pro
            260                 265                 270

Ala Val Lys Gly Lys Pro Leu Asn Asp Leu Leu Asn Lys Ala Ile Leu
        275                 280                 285

Asp Gly Ala Thr Ile Asp Asp Leu Gln Thr Ile Glu Lys Glu Trp Leu
    290                 295                 300

Ala Lys Ala Asp Val Lys Leu Phe His Glu Val Phe Ala Asp Ala Ala
305                 310                 315                 320

Lys Ala Ala Gly Lys Asp Gln Ser Val Ile Asp Gln Phe Asn Ser Lys
                325                 330                 335

Val Asn Pro Leu Ser Glu Thr Ser Ile Tyr Glu Met Gln Ala Leu Ala
            340                 345                 350

Lys Glu Leu Leu Gly Thr Glu Leu Phe Phe Asp Trp Asp Leu Pro Arg
        355                 360                 365

Gly Arg Glu Gly Leu Tyr Arg Tyr Gln Gly Gly Thr Gln Cys Ser Val

370                 375                 380

Met Arg Ala Arg Ala Phe Ala Pro Tyr Ala Asp Leu Cys Trp Met Glu
385                 390                 395                 400

Ser Asn Tyr Pro Asp Tyr Glu Gln Ala Lys Glu Phe Ala Glu Gly Val
                405                 410                 415

Thr Ala Lys Phe Pro Gly Lys Trp Met Ala Tyr Asn Leu Ser Pro Ser
            420                 425                 430

Phe Asn Trp Thr Lys Ala Met Ser Val Asp Glu Gln Glu Thr Phe Ile
        435                 440                 445

Gln Arg Leu Gly Asp Leu Gly Tyr Ile Trp Gln Phe Ile Thr Leu Ala
    450                 455                 460

Gly Leu His Thr Ser Gly Leu Ala Ile Glu Gln Phe Ser Lys Asn Phe
465                 470                 475                 480

Ala Lys Leu Gly Met Lys Ala Tyr Ala Gln Asp Ile Gln Lys Lys Glu
                485                 490                 495

Leu Asp Asn Gly Ile Asp Met Val Lys His Gln Lys Trp Ser Gly Ala
            500                 505                 510

Glu Tyr Ile Asp Gly Leu Leu Arg Leu Ala Gln Gly Gly Leu Ala Ala
        515                 520                 525

Thr Ala Ala Met Gly Gln Gly Val Thr Glu Asp Gln Phe Lys
    530                 535                 540

<210>  19
<211>  551
<212>  PRT
<213>  Artificial Sequence

<220>
<223>  Sacharomyces cerevisiae malate synthase amino acid sequence
       (artificial)
       Saccharomyces cerevisiae peroxisomal malate synthase (Mls1) amino
       acid sequence, lacking the 3 C-terminal peroxisomal targeting

<400>  19

Met Val Lys Val Ser Leu Asp Asn Val Lys Leu Leu Val Asp Val Asp
1               5                   10                  15

Lys Glu Pro Phe Phe Lys Pro Ser Ser Thr Thr Val Gly Asp Ile Leu
            20                  25                  30

Thr Lys Asp Ala Leu Glu Phe Ile Val Leu Leu His Arg Thr Phe Asn
        35                  40                  45

Asn Lys Arg Lys Gln Leu Leu Glu Asn Arg Gln Val Val Gln Lys Lys
    50                  55                  60

Leu Asp Ser Gly Ser Tyr His Leu Asp Phe Leu Pro Glu Thr Ala Asn
65                  70                  75                  80

Ile Arg Asn Asp Pro Thr Trp Gln Gly Pro Ile Leu Ala Pro Gly Leu
                85                  90                  95

Ile Asn Arg Ser Thr Glu Ile Thr Gly Pro Pro Leu Arg Asn Met Leu
            100                 105                 110

Ile Asn Ala Leu Asn Ala Pro Val Asn Thr Tyr Met Thr Asp Phe Glu
        115                 120                 125

Asp Ser Ala Ser Pro Thr Trp Asn Asn Met Val Tyr Gly Gln Val Asn
    130                 135                 140

Leu Tyr Asp Ala Ile Arg Asn Gln Ile Asp Phe Asp Thr Pro Arg Lys
145                 150                 155                 160

Ser Tyr Lys Leu Asn Gly Asn Val Ala Asn Leu Pro Thr Ile Ile Val
                165                 170                 175

Arg Pro Arg Gly Trp His Met Val Glu Lys His Leu Tyr Val Asp Asp
            180                 185                 190

Glu Pro Ile Ser Ala Ser Ile Phe Asp Phe Gly Leu Tyr Phe Tyr His
        195                 200                 205

Asn Ala Lys Glu Leu Ile Lys Leu Gly Lys Gly Pro Tyr Phe Tyr Leu
    210                 215                 220

Pro Lys Met Glu His His Leu Glu Ala Lys Leu Trp Asn Asp Val Phe

225                 230                 235                 240

Cys Val Ala Gln Asp Tyr Ile Gly Ile Pro Arg Gly Thr Ile Arg Ala
                245                 250                 255

Thr Val Leu Ile Glu Thr Leu Pro Ala Ala Phe Gln Met Glu Glu Ile
            260                 265                 270

Ile Tyr Gln Leu Arg Gln His Ser Ser Gly Leu Asn Cys Gly Arg Trp
        275                 280                 285

Asp Tyr Ile Phe Ser Thr Ile Lys Arg Leu Arg Asn Asp Pro Asn His
    290                 295                 300

Ile Leu Pro Asn Arg Asn Gln Val Thr Met Thr Ser Pro Phe Met Asp
305                 310                 315                 320

Ala Tyr Val Lys Arg Leu Ile Asn Thr Cys His Arg Arg Gly Val His
                325                 330                 335

Ala Met Gly Gly Met Ala Ala Gln Ile Pro Ile Lys Asp Asp Pro Ala
            340                 345                 350

Ala Asn Glu Lys Ala Met Thr Lys Val Arg Asn Asp Lys Ile Arg Glu
        355                 360                 365

Leu Thr Asn Gly His Asp Gly Ser Trp Val Ala His Pro Ala Leu Ala
    370                 375                 380

Pro Ile Cys Asn Glu Val Phe Ile Asn Met Gly Thr Pro Asn Gln Ile
385                 390                 395                 400

Tyr Phe Ile Pro Glu Asn Val Val Thr Ala Ala Asn Leu Leu Glu Thr
                405                 410                 415

Lys Ile Pro Asn Gly Glu Ile Thr Thr Glu Gly Ile Val Gln Asn Leu
            420                 425                 430

Asp Ile Gly Leu Gln Tyr Met Glu Ala Trp Leu Arg Gly Ser Gly Cys
        435                 440                 445

Val Pro Ile Asn Asn Leu Met Glu Asp Ala Ala Thr Ala Glu Val Ser
    450                 455                 460

Arg Cys Gln Leu Tyr Gln Trp Val Lys His Gly Val Thr Leu Lys Asp
465                 470                 475                 480

Thr Gly Glu Lys Val Thr Pro Glu Leu Thr Glu Lys Ile Leu Lys Glu
                485                 490                 495

Gln Val Glu Arg Leu Ser Lys Ala Ser Pro Leu Gly Asp Lys Asn Lys
            500                 505                 510

Phe Ala Leu Ala Ala Lys Tyr Phe Leu Pro Glu Ile Arg Gly Glu Lys
        515                 520                 525

Phe Ser Glu Phe Leu Thr Thr Leu Leu Tyr Asp Glu Ile Val Ser Thr
    530                 535                 540

Lys Ala Thr Pro Thr Asp Leu
545                 550